# EUROPEAN PATENT APPLICATION

(11) **EP 3 922 695 A1**
(43) Date of publication of application: **15.12.2021**
(21) Application number: 20752308.5
(22) Date of filing: 07.02.2020
(51) Int. Cl.: C09K 11/06, H01L 51/50

(54) **ORGANIC ELECTROLUMINESCENT DEVICE**

(30) Priority: 07.02.2019 JP 2019020559
(71) Applicant: Hodogaya Chemical Co., Ltd., Tokyo 104-0028 (JP)
(72) Inventor: SURUGA, Kazuyuki, Tokyo 104-0028 (JP); YAMAMOTO, Takeshi, Tokyo 104-0028 (JP); MOCHIZUKI, Shunji, Tokyo 104-0028 (JP); HIRAYAMA, Yuta, Tokyo 104-0028 (JP); HAYASHI, Shuichi, Tokyo 104-0028 (JP)
(74) Representative: Müller-Boré & Partner Patentanwälte PartG mbB
(86) International application number: PCT/JP2020/004806
(87) International publication number: WO 2020/162594

(57) **Abstract**

[Object] An object of the present invention is to provide , as a material for an organic EL device having high efficiency and a high durability, a material for an organic EL device having excellent injection/transport performance of holes, excellent electron blocking capability, excellent stability in a thin-film state, and an excellent durability, and to further provide an organic EL device having high efficiency, a low drive voltage, and a long lifetime by combining the material and various materials for an organic EL device having excellent injection/transport performance of holes and electrons, excellent electron blocking capability, excellent stability in a thin-film state, and an excellent durability so that the properties of the respective materials can be effectively exhibited.

[Solving Means] Since an arylamine compound having a specific structure in the present invention has excellent injection/transport performance of holes, excellent stability in a thin-film state, and an excellent durability, holes injected from the anode side can be effectively transported by selecting this arylamine compound having a specific structure as a material of a hole transport layer. Further, various organic EL devices obtained by combining an electron transport material having a specific structure and the like exhibit favorable device properties.

## Description

### Technical Field

The present invention relates to an organic electroluminescence device that is a self-light-emitting device suitable for various display devices, and specifically to an organic electroluminescence device (hereinafter, abbreviated as an organic EL device) that uses a specific arylamine compound.

### Background Art

Since the organic EL device is a self-light-emitting device, it is brighter than the liquid crystal device and excellent in visibility and capable of performing clear display, and thus, active research has been done thereon.

In 1987, C.W.Tang et al. (Eastman Kodak Company) have developed a stacked-structure device in which various roles are assigned to the materials, and put an organic EL device using an organic material to practical use. They have stacked a fluorophore capable of transporting electrons and an organic matter capable of transporting holes, and injected both charges into a fluorophore layer to emit light, thereby achieving high luminance of 1000 cd/m² or more with a voltage of 10 V or less (see, for example, Patent Literature 1 and Patent Literature 2).

Many improvements have been made for practical use of the organic EL device until now. In an electroluminescence device that subdivides the various roles and includes an anode, a hole injection layer, a hole transport layer, a light-emitting layer, an electron transport layer, an electron injection layer, and a cathode in the stated order on a substrate, high efficiency and durability have been achieved (see, for example, Non-Patent Literature 1).

Further, for the purpose of further improving the light emission efficiency, attempts have been made to use a triplet exciton and utilization of a phosphorescent compound is being considered (see, for example, Non-Patent Literature 2).

Then, also a device utilizing light emission by thermally activated delayed fluorescence (TADF) has been developed. In 2011, Atachi et al. (Kyushu University) have achieved an external quantum efficiency of 5.3% by a device using a thermally activated delayed fluorescence material (see, for example, Non-Patent Literature 3).

The light-emitting layer can also be prepared by doping a charge transport compound generally called a host material with a fluorescent compound, a phosphorescent compound, or a material emitting delayed fluorescence. As described in the Non-Patent Literature, selection of an organic material in an organic EL device greatly affects various properties such as the efficiency and durability of the device (see, for example, Non-Patent Literature 2).

In an organic EL device, charges injected from both electrodes are recombined in the light-emitting layer to obtain light emission. It is important how to efficiently deliver both charges of holes and electrons to the light-emitting layer, and it is necessary to make a device excellent in carrier balance. Further, it is possible to achieve high light emission efficiency by increasing the hole injection property, improving the electron blocking performance for blocking electrons injected from a cathode to improve the probability of recombination of holes and electrons, and confining excitons generated in the light-emitting layer. For this reason, the role played by a hole transport material is important, and there is a need for a hole transport material having a high hole injection property, a high mobility of holes, high electron blocking performance, and a high durability for electrons.

Further, regarding the device lifetime, the heat resistance and amorphous property of the material are also important. In a material having a low heat resistance, thermal decomposition occurs even at a low temperature due to the heat generated when the device is driven, and the material deteriorates. In a material having a low amorphous property, the thin film is crystallized even in a short time, and the device deteriorates. For this reason, the material to be used needs to have properties of a high heat resistance and a favorable amorphous property.

As the hole transport material that has been used for an organic EL device, N,N'-diphenyl-N,N'-di(α-naphthyl)benzidine (NPD) and various aromatic amine derivatives have been known (see, for example, Patent Literature 1 and Patent Literature 2). NPD has a favorable hole transport capacity, but the glass transition point (Tg), which is an index of a heat resistance, thereof is low, i.e., 96°C, resulting in the deterioration of device properties due to crystallization under high-temperature conditions (see, for example, Non-Patent Literature 4). Further, among the aromatic amine derivatives described in the Patent Literatures, compounds having an excellent mobility of 10⁻³ cm²/Vs or more have been known (see, for example, Patent Literature 1 and Patent Literature 2), but the electron blocking performance thereof is insufficient. For this reason, some electrons pass through the light-emitting layer, and improvement in the light emission efficiency cannot be expected. In order to achieve higher efficiency, there has been a need for a material having higher electron blocking performance, higher stability in a thin-film state, and a high heat resistance. Further, an aromatic amine derivative having a high durability is reported (see, for example, Patent Literature 3). However, the aromatic amine derivative is used as a charge transport material used for a xerographic photoreceptor, and there has been no example of using it as an organic EL device.

As a compound having improved properties such as a heat resistance and a hole injection property, an arylamine compound having a substituted carbazole structure is proposed (see, for example, Patent Literature 4 and Patent Literature 5). A device using one of these compounds for a hole injection layer or a hole transport layer has an improved heat resistance, improved light emission efficiency, and the like, but they are not yet sufficient. Therefore, a lower drive voltage and higher light emission efficiency are desired.

In order to improve the device properties of an organic EL device and improve the yield of device preparation, there is a need for a device having high light emission efficiency, a low drive voltage, and a long lifetime, in which holes and electrons can be recombined with each other with high efficiency, by combining materials having excellent injection/transport performance of holes and electrons, excellent stability in a thin-film state, and an excellent durability.

Further, in order to improve the device properties of an organic EL device, there is a need for a device having better carrier balance, high efficiency, a low drive voltage, and a long lifetime by combining materials having excellent injection/transport performance of holes and electrons, excellent stability in a thin-film state, and an excellent durability.

### Citation List

### Patent Literature

Patent Literature 1: Japanese Patent Application Laid-open No. 1996-048656
Patent Literature 2: Japanese Patent No. 3194657
Patent Literature 3: Japanese Patent No. 4943840
Patent Literature 4: Japanese Patent Application Laid-open No. 2006-151979
Patent Literature 5: WO 2008/62636
Patent Literature 6: Korean Published Patent No. 10-2013-0060157
Patent Literature 7: Korean Published Patent No. 10-2015-0130206
Patent Literature 8: WO 2014/009310

### Non-Patent Literature

Non-Patent Literature 1: The Japan Society of Applied Physics, proceedings of the ninth workshop, pp. 55-61 (2001)
Non-Patent Literature 2: The Japan Society of Applied Physics, proceedings of the ninth workshop, pp. 23-31 (2001)
Non-Patent Literature 3:
   Appl.Phys.Let.,98,083302(2011)
Non-Patent Literature 4: The 3rd Meeting of the Japan OLED Forum, the book of abstracts, pp. 13 to 14 (2006)

### Disclosure of Invention

### Technical Problem

An object of the present invention is to provide, as a material for an organic EL device having high efficiency and a high durability, a material for an organic EL device having excellent injection/transport performance of holes, excellent electron blocking capability, excellent stability in a thin-film state, and an excellent durability, and to further provide an organic EL device having high efficiency, a low drive voltage, and a long lifetime by combining the material and various materials for an organic EL device having excellent injection/transport performance of holes and electrons, excellent electron blocking capability, excellent stability in a thin-film state, and an excellent durability so that the properties of the respective materials can be effectively exhibited.

Examples of the physical properties that an organic compound to be provided by the present invention should have include (1) having a high hole injection property, (2) having a high mobility of holes, (3) having high stability in a thin-film state, and (4) having an excellent heat resistance. Further, examples of the physical properties that an organic EL device to be provided by the present invention should have include (1) having high light emission efficiency and high power efficiency, (2) having a low light emission start voltage, (3) having a low practical driving voltage, and (4) having a long lifetime.

### Solution to Problem

In this regard, in order to achieve the above-mentioned object, the present inventors have selected various arylamine compounds to prepare an organic EL device, focusing on the fact that an arylamine compound having a specific structure has excellent hole injection/transport performance, excellent stability in a thin-film state, and an excellent durability, and the properties of the device were intensively evaluated. As a result, the present inventors have found that holes injected from the side of an anode can be efficiently transported by selecting an arylamine compound having a specific structure as a material of a hole transport layer. Further, the present inventors have prepared various organic EL devices obtained by combining an electron transport material having a specific structure and the like and intensively evaluated the properties of each of the device. As a result, the present invention was completed.

That is, in accordance with the present invention, the following organic EL device is provided.

1) An organic electroluminescence device, including at least an anode, a hole transport layer, a light-emitting layer, an electron transport layer, and a cathode in the stated order, the organic EL device being characterized in that the hole transport layer includes an arylamine compound represented by the following general formula (1).

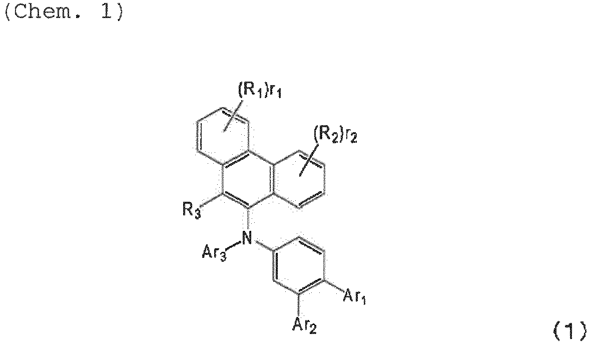

(In the formula, R₁ and R₂ each represent a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. R₃ represents a hydrogen atom, a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. Ar₁ to Ar₃ each represent a substituted or unsubstituted aromatic hydrocarbon group, a substituted or unsubstituted aromatic heterocyclic group, or a substituted or unsubstituted fused polycyclic aromatic group. r₁ and r₂ may be the same as or different from each other and each represent an integer of 0 to 4.)

2) The organic EL device according to 1) above, characterized in that the arylamine compound is represented by the following general formula (1a).

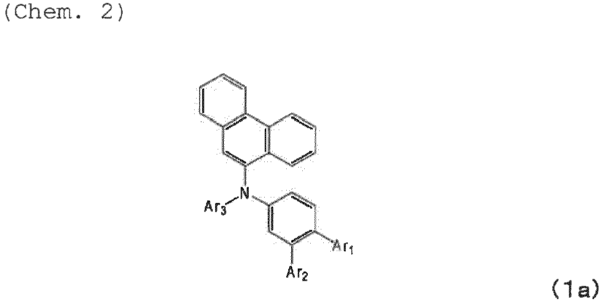

(In the formula, Ar₁ to Ar₃ are as shown in the general formula (1).)

3) The organic EL device according to 2) above, characterized in that the Ar3 is a substituted or unsubstituted phenyl group.

4) The organic EL device according to any one of 1) to 3) above, characterized in that the electron transport layer includes a compound having a pyrimidine ring structure represented by the following general formula (2).

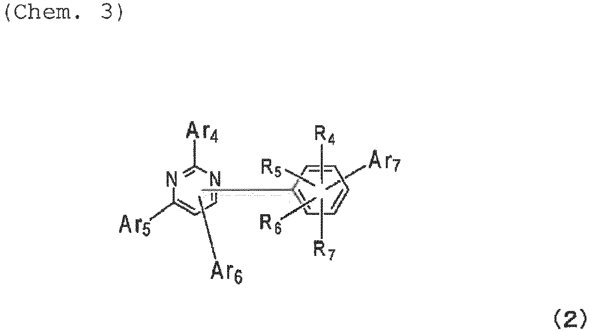

(In the formula, Ar₄ represents a substituted or unsubstituted aromatic hydrocarbon group or a substituted or unsubstituted fused polycyclic aromatic group, Ar₅ and Ar₆ may be the same as or different from each other and each represent a hydrogen atom, a substituted or unsubstituted aromatic hydrocarbon group, or a substituted or unsubstituted fused polycyclic aromatic group, Ar₇ represents a substituted or unsubstituted aromatic heterocyclic group, and R₄ to R₇ may be the same as or different from each other and each represent a hydrogen atom, a deuterium atom, a fluorine atom, a chlorine atom, a cyano group, a trifluoromethyl group, a linear or branched alkyl group having 1 to 6 carbon atoms, a substituted or unsubstituted aromatic hydrocarbon group, a substituted or unsubstituted aromatic heterocyclic group, or a substituted or unsubstituted fused polycyclic aromatic group. Here, there is no case that both Ar₅ and Ar₆ are hydrogen atoms.)

5) The organic EL device according to any one of 1) to 4) above, characterized in that the hole transport layer has a two-layer structure of a first hole transport layer and a second hole transport layer, and the second hole transport layer is adjacent to the light-emitting layer and includes the arylamine compound.

6) The organic EL device according to 5) above, characterized in that the first hole transport layer includes a triphenylamine derivative different from the arylamine compound included in the second hole transport layer, and the triphenylamine derivative is a compound that has a molecular structure in which two triphenylamine skeletons are linked to each other by a single bond or a divalent hydrocarbon group and has two to six triphenylamine skeletons as a whole molecule.

7) The organic EL device according to 5) or 6) above characterized in that the triphenylamine derivative included in the first hole transport layer is a compound represented by the following general formula (3).

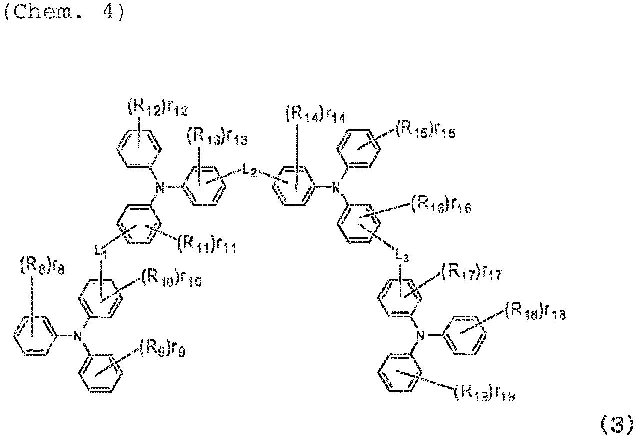

(In the formula, R₈ to R₁₉ each represent a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₈ to r₁₉ may be the same as or different from each other, r₈, r₉, r₁₂, r₁₅, r₁₈, and r₁₉ each represent an integer of 0 or 1 to 5, and r₁₀, r₁₁, r₁₃, r₁₄, r₁₆, and r₁₇ each represent an integer of 0 or 1 to 4. In the case where r₈, r₉, r₁₂, r₁₅, r₁₈, and r₁₉ each represent an integer of 2 to 5 or r₁₀, r₁₁, r₁₃, r₁₄, r₁₆, and r₁₇ each represent an integer of 2 to 4, a plurality of R₈, a plurality of R₉, a plurality of R₁₀, a plurality of R₁₁, a plurality of R₁₂, a plurality of R₁₃, a plurality of R₁₄, a plurality of R₁₅, a plurality of R₁₆, a plurality of R₁₇, a plurality of R₁₈, or a plurality of R₁₉ bonded to the same benzene ring may be the same as or different from each other and may be bonded to each other via a single bond, a substituted or unsubstituted methylene group, an oxygen atom, or a sulfur atom to form a ring. L₁, L₂, and L₃ may be the same as or different from each other and each represent a divalent group represented by one of the following structural formulae (B) to (G) or a single bond.) 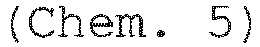 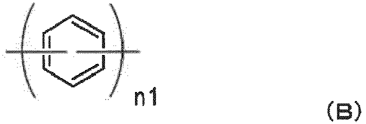 (In the formula, n1 represents an integer of 1 to 3.)

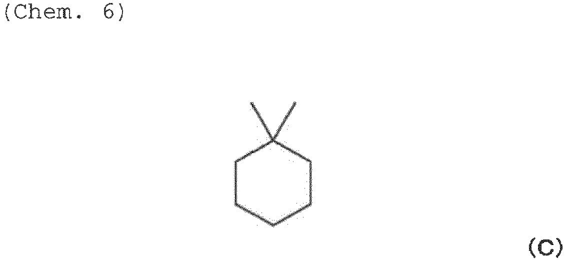

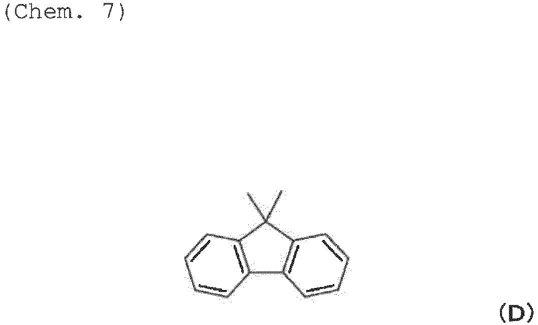

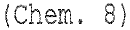

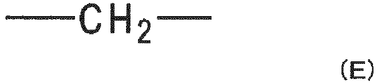

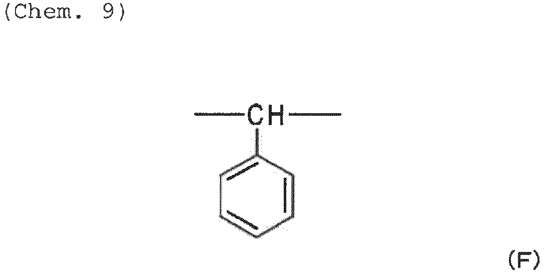

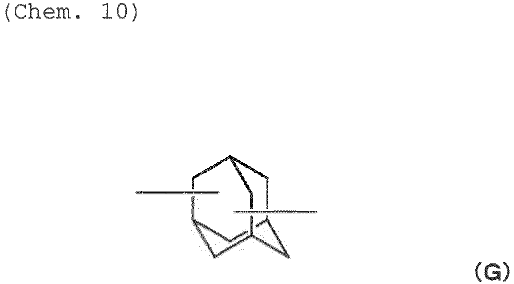

8) The organic EL device according to 5) or 6) above, characterized in that the triphenylamine derivative included in the first hole transport layer is a compound represented by the following general formula (4).

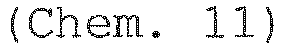

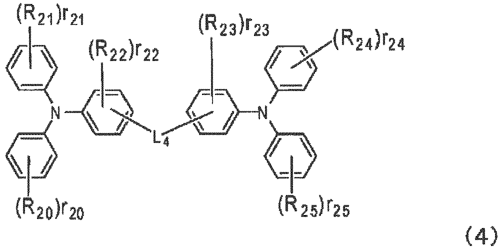

(In the formula, R₂₀ to R₂₅ each represent a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₂₀ to r₂₅ may be the same as or different from each other, r₂₀, r₂₁, r₂₄, and r₂₅ each represent an integer of 0 or 1 to 5, and r₂₂ and r₂₃ each represent an integer of 0 or 1 to 4. In the case where r₂₀, r₂₁, r₂₄, and r₂₅ each represent an integer of 2 to 5 or r₂₂ and r₂₃ each represent an integer of 2 to 4, a plurality of R₂₀, a plurality of R₂₁, a plurality of R₂₂, a plurality of R₂₃, a plurality of R₂₄, or a plurality of R₂₅ bonded to the same benzene ring may be the same as or different from each other and may be bonded to each other via a single bond, a substituted or unsubstituted methylene group, an oxygen atom, or a sulfur atom to form a ring. L₄ represents a divalent group represented by one of the following structural formulae (B) to (G) or a single bond.) 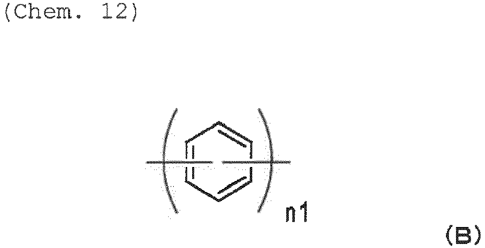 (In the formula, n1 represents an integer of 1 to 3.) 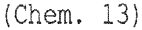 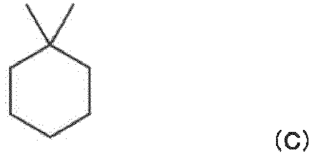 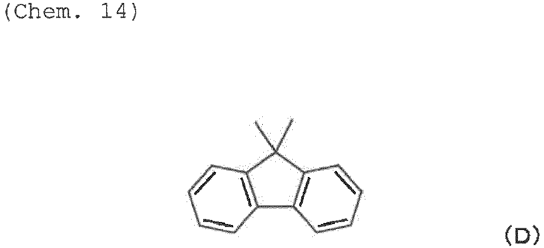 (Chem. 15)

-CH₂- (E)

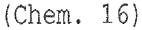

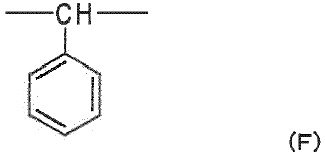

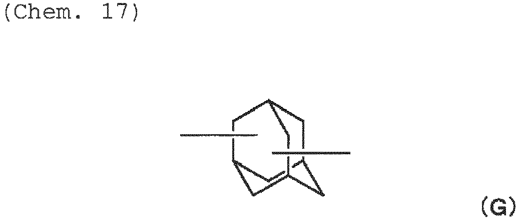

9) The organic EL device according to any one of 1) to 8) above, characterized in that the light-emitting layer includes a blue luminescent dopant.

10) The organic EL device according to 9) above, characterized in that the blue luminescent dopant is a pyrene derivative having a pyrene skeleton in a molecule.

11) The organic EL device according to 9) above, characterized in that the blue luminescent dopant is an amine derivative having a fused ring structure represented by the following general formula (5).

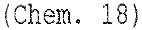

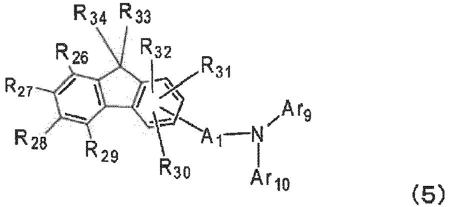

(In the formula, A₁ represents a divalent group of a substituted or unsubstituted aromatic hydrocarbon, a divalent group of a substituted or unsubstituted aromatic heterocyclic ring, a divalent group of a substituted or unsubstituted fused polycyclic aromatic, or a single bond, and Arg and Ar₁₀ may be the same as or different from each other, each represent a substituted or unsubstituted aromatic hydrocarbon group, a substituted or unsubstituted aromatic heterocyclic group, or a substituted or unsubstituted fused polycyclic aromatic group, and may be bonded to each other via a single bond, a substituted or unsubstituted methylene group, an oxygen atom, or a sulfur atom to form a ring. R₂₆ to R₂₉ may be the same as or different from each other and each represent a hydrogen atom, a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, a substituted or unsubstituted aryloxy group, or a disubstituted amino group substituted with a group selected from the group consisting of an aromatic hydrocarbon group, an aromatic heterocyclic group, and a fused polycyclic aromatic group, and R₂₆ to R₂₉ may be bonded to each other via a single bond, a substituted or unsubstituted methylene group, an oxygen atom, or a sulfur atom to form a ring and may be bonded to the benzene ring to which R₂₆ to R₂₉ are bonded via a linking group such as a substituted or unsubstituted methylene group, an oxygen atom, a sulfur atom, and a monosubstituted amino group to form a ring. R₃₀ to R₃₂ may be the same as or different from each other and each represent a hydrogen atom, a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and t R₃₀ to R₃₂ may be bonded to each other via a single bond, a substituted or unsubstituted methylene group, an oxygen atom, or a sulfur atom to form a ring and may be bonded to the benzene ring to which R₃₀ to R₃₂ are bonded via a linking group such as a substituted or unsubstituted methylene group, an oxygen atom, a sulfur atom, and a monosubstituted amino group to form a ring. R₃₃ and R₃₄ may be the same as or different from each other and each represent a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and R₃₃ and R₃₄ may be bonded to each other via a linking group such as a single bond, a substituted or unsubstituted methylene group, an oxygen atom, a sulfur atom, and a monosubstituted amino group to form a ring.)

12) The organic EL device according to any one of 1) to 11) above, characterized in that the light-emitting layer includes an anthracene derivative having an anthracene skeleton in a molecule.

13) The organic EL device according to 12) above, characterized in that the light-emitting layer includes a host material that is an anthracene derivative.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₁ to R₃ in the general formula (1) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group.

Specific examples of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1) include a deuterium atom, a cyano group, a nitro group; a halogen atom such as a fluorine atom, a chlorine atom, a bromine atom, and an iodine atom; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; a disubstituted amino group substituted with an aromatic hydrocarbon group or a fused polycyclic aromatic group, such as a diphenylamino group and a dinaphthylamino group; a disubstituted amino group substituted with an aromatic heterocyclic group, such as a dipyridylamino group and a dithienylamino group; and a disubstituted amino group substituted with a substituent group selected from the group consisting of an aromatic hydrocarbon group, a fused polycyclic aromatic group, and an aromatic heterocyclic group. These substituent groups may be further substituted with the exemplified substituted groups.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₁ to R₃ in the general formula (1) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1).

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₁ to R₃ in the general formula (1) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1).

Specific examples of the "aryloxy group" in the "substituted or unsubstituted aryloxy group" represented by R₁ to R₃ in the general formula (1) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1).

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by Ar₁ to Ar₃ in the general formula (1) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1).

Specific examples of the "aromatic hydrocarbon group" or "fused polycyclic aromatic group" in the "substituted or unsubstituted aromatic hydrocarbon group" or "substituted or unsubstituted fused polycyclic aromatic group" represented by Ar₄ to Ar₆ in the general formula (2) include a phenyl group, a biphenylyl group, a terphenylyl group, a quarterphenyl group, a styryl group, a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1). Further, these substituent groups may be bonded to each other via a single bond, a substituted or unsubstituted methylene group, an oxygen atom, or a sulfur atom to form a ring.

Specific examples of the "aromatic heterocyclic group" in the "substituted or unsubstituted aromatic heterocyclic group" represented by Ar₇ in the general formula (2) include a triazinyl group, a pyridyl group, a pyrimidinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1). Further, these substituent groups may be bonded to each other via a single bond, a substituted or unsubstituted methylene group, an oxygen atom, or a sulfur atom to form a ring.

Specific examples of the "linear or branched alkyl group having 1 to 6 carbon atoms" represented by R₄ to R₇ in the general formula (2) include a methyl group, an ethyl group, an n-propyl group, an isopropyl group, an n-butyl group, a 2-methyl propyl group, a t-butyl group, an n-pentyl group, a 3-methylbutyl group, a tert-pentyl group, an n-hexyl group, an iso-hexyl group, and a tert-hexyl group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1). Further, these substituent groups may be bonded to each other via a single bond, a substituted or unsubstituted methylene group, an oxygen atom, or a sulfur atom to form a ring.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₄ to R₇ in the general formula (2) include a phenyl group, a biphenylyl group, a terphenylyl group, a quarterphenyl group, a styryl group, a naphthyl group, an anthracenyl group, an acenaphthenyl group, a phenanthrenyl group, a fluorenyl group, an indenyl group, a pyrenyl group, a perylenyl group, a fluoranthenyl group, a triphenylenyl group, a triazinyl group, a pyridyl group, a pyrimidinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₁ to R₃ in the general formula (1). Further, these substituent groups may be bonded to each other via a single bond, a substituted or unsubstituted methylene group, an oxygen atom, or a sulfur atom to form a ring.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₈ to R₁₉ in the general formula (3) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group. Further, in the case where a plurality of groups including these groups is bonded to the same benzene ring (r₈, r₉, r₁₂, r₁₅, r₁₈, and r₁₉ each represent an integer of 2 to 5 or r₁₀, r₁₁, r₁₃, r₁₄, r₁₆, and r₁₇ each represent an integer of 2 to 4), the plurality of groups may be bonded to each other via a single bond, a substituted or unsubstituted methylene group, an oxygen atom, or a sulfur atom to form a ring.

Specific examples of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) include a deuterium atom, a cyano group, a nitro group; a halogen atom such as a fluorine atom, a chlorine atom, a bromine atom, and an iodine atom; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; and an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group. These substituent groups may be further substituted with the exemplified substituted groups. Further, these substituent groups may be bonded to each other via a single bond, a substituted or unsubstituted methylene group, an oxygen atom, or a sulfur atom to form a ring.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₈ to R₁₉ in the general formula (3) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. Further, in the case where a plurality of groups including these groups is bonded to the same benzene ring (r₈, r₉, r₁₂, r₁₅, r₁₈, and r₁₉ each represent an integer of 2 to 5 or r₁₀, r₁₁, r₁₃, r₁₄, r₁₆, and r₁₇ each represent an integer of 2 to 4), the plurality of groups may be bonded to each other via a single bond, a substituted or unsubstituted methylene group, an oxygen atom, or a sulfur atom to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₈ to R₁₉ in the general formula (3) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group. Further, in the case where a plurality of groups including these groups is bonded to the same benzene ring (r₈, r₉, r₁₂, r₁₅, r₁₈, and r₁₉ each represent an integer of 2 to 5 or r₁₀, r₁₁, r₁₃, r₁₄, r₁₆, and r₁₇ each represent an integer of 2 to 4), the plurality of groups may be bonded to each other via a single bond, a substituted or unsubstituted methylene group, an oxygen atom, or a sulfur atom to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Specific examples of the "aryloxy group" in the "substituted or unsubstituted aryloxy group" represented by R₈ to R₁₉ in the general formula (3) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group. Further, in the case where a plurality of groups including these groups is bonded to the same benzene ring (r₈, r₉, r₁₂, r₁₅, r₁₈, and r₁₉ each represent an integer of 2 to 5 or r₁₀, r₁₁, r₁₃, r₁₄, r₁₆, and r₁₇ each represent an integer of 2 to 4), the plurality of groups may be bonded to each other via a single bond, a substituted or unsubstituted methylene group, an oxygen atom, or a sulfur atom to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

In the general formula (3), r₈ to r₁₉ may be the same as or different from each other, r₈, r₉, r₁₂, r₁₅, r₁₈, and r₁₉ each represent an integer of 0 or 1 to 5, and r₁₀, r₁₁, r₁₃, r₁₄, r₁₆, and r₁₇ each represent an integer of 0 or 1 to 4. In the case where r₈, r₉, r₁₀, r₁₁, r₁₂, r₁₃, r₁₄, r₁₅, r₁₆, r₁₇, r₁₈, or r₁₉ represents 0, it represents that r₈, r₉, r₁₀, r₁₁, r₁₂, r₁₃, r₁₄, r₁₅, r₁₆, r₁₇, r₁₈, or r₁₉ is not present on a benzene ring, i.e., the benzene ring is not substituted with the group represented by r₈, r₉, r₁₀, r₁₁, r₁₂, r₁₃, r₁₄, r₁₅, r₁₆, r₁₇, r₁₈, or r₁₉.

In the structural formula (B) of the general formula (3), n1 represents an integer of 1 to 3.

Examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₂₀ to R₂₅ in the general formula (4) include the similar ones as described for the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₂₀ to R₂₅ in the general formula (4) include the similar ones as described for the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₈ to R₁₉ in the general formula (3), and aspects similar to those of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₈ to R₁₉ in the general formula (3) can be taken.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₂₀ to R₂₅ in the general formula (4) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₈ to R₁₉ in the general formula (3). Further, in the case where a plurality of groups including these groups is bonded to the same benzene ring (r₁₇, r₁₈, r₂₁, and r₂₂ each represent an integer of 2 to 5 or r₁₉ and r₂₀ each represent an integer of 2 to 4), the plurality of groups may be bonded to each other via a single bond, a substituted or unsubstituted methylene group, an oxygen atom, or a sulfur atom to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aryloxy group" in the "substituted or unsubstituted aryloxy group" represented by R₂₀ to R₂₅ in the general formula (4) include the similar ones as described for the "aryloxy group" in the "substituted or unsubstituted aryloxy group" represented by R₈ to R₁₉ in the general formula (3), and aspects similar to those of the "aryloxy group" in the "substituted or unsubstituted aryloxy group" represented by R₈ to R₁₉ in the general formula (3) can be taken.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

In the general formula (4), r₂₀ to r₂₅ may be the same as or different from each other, r₂₀, r₂₁, r₂₄, and r₂₅ each represent an integer of 0 or 1 to 5, and r₂₂ and r₂₃ each represent an integer of 0 or 1 to 4. In the case where r₂₀, r₂₁, r₂₂, r₂₃, r₂₄, or r₂₅ represents 0, it represents that r₂₀, r₂₁, r₂₂, r₂₃, r₂₄, or r₂₅ is not present on a benzene ring, i.e., the benzene ring is not substituted with the group represented by r₂₀, r₂₁, r₂₂, r₂₃, r₂₄, or r₂₅.

In the structural formula (B) of the general formula (4), n1 represents an integer of 1 to 3.

Specific examples of the "aromatic hydrocarbon", "aromatic heterocyclic ring", or "fused polycyclic aromatic" of the "substituted or unsubstituted aromatic hydrocarbon", "substituted or unsubstituted aromatic heterocyclic ring", or "substituted or unsubstituted fused polycyclic aromatic" in the "divalent group of a substituted or unsubstituted aromatic hydrocarbon", "divalent group of a substituted or unsubstituted aromatic heterocyclic ring", or "divalent group of a substituted or unsubstituted fused polycyclic aromatic" represented by A₁ in the general formula (5) include benzene, biphenyl, terphenyl, tetrakisphenyl, styrene, naphthalene, anthracene, acenaphthalene, fluorene, phenanthrene, indan, pyrene, triphenylene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

Then, the "divalent group of a substituted or unsubstituted aromatic hydrocarbon", "divalent group of a substituted or unsubstituted aromatic heterocyclic ring", or "divalent group of a substituted or unsubstituted fused polycyclic aromatic" represented by A₁ in the general formula (5) represents a divalent group formed by removing two hydrogen atoms from the "aromatic hydrocarbon", "aromatic heterocyclic ring", or "fused polycyclic aromatic" described above.

Further, these divalent group may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by Ar₉ and Ar₁₀ in the general formula (5) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₈ to R₁₉ in the general formula (3), and Ar₉ and Ar₁₀ may be bonded to each other via a single bond, a substituted or unsubstituted methylene group, an oxygen atom, or a sulfur atom to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₂₆ to R₃₄ in the general formula (5) include the similar ones as described for the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₈ to R₁₉ in the general formula (3), and these groups may be bonded to each other via a linking group such as a single bond, a substituted or unsubstituted methylene group, an oxygen atom, a sulfur atom, and a monosubstituted amino group to form a ring.

Further, these groups may each have a substituent group. Examples of the substituent group include the similar ones as described for the "substituent group" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₈ to R₁₉ in the general formula (3), and aspects similar to those of the "substituent group" in the "linear or branched alkyl group having 1 to 6 carbon atoms which may have a substituent group", "cycloalkyl group having 5 to 10 carbon atoms which may have a substituent group", or "linear or branched alkenyl group having 2 to 6 carbon atoms which may have a substituent group" represented by R₈ to R₁₉ in the general formula (3) can be taken.

Examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₂₆ to R₃₂ in the general formula (5) include the similar ones as described for the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituent group" or "cycloalkyloxy group having 5 to 10 carbon atoms which may have a substituent group" represented by R₈ to R₁₉ in the general formula (3), and these groups may be bonded to each other via a linking group such as a single bond, a substituted or unsubstituted methylene group, an oxygen atom, a sulfur atom, and a monosubstituted amino group to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₂₆ to R₃₂ in the general formula (5) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₈ to R₁₉ in the general formula (3), and these groups may be bonded to each other via a single bond, a substituted or unsubstituted methylene group, an oxygen atom, or a sulfur atom to form a ring. These groups (R₂₆ to R₃₂) and the benzene ring(s) to which the groups (R₂₆ to R₃₂) are directly bonded may be bonded to each other via a linking group such as a substituted or unsubstituted methylene group, an oxygen atom, a sulfur atom, and a monosubstituted amino group to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₃₃ and R₃₄ in the general formula (5) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₈ to R₁₉ in the general formula (3), and these groups may be bonded to each other via a linking group such as a single bond, a substituted or unsubstituted methylene group, an oxygen atom, a sulfur atom, and a monosubstituted amino group to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aryloxy group" in the "substituted or unsubstituted aryloxy group" represented by R₂₆ to R₃₄ in the general formula (5) include the similar ones as described for the "aryloxy group" in the "aryloxy group" represented by R₈ to R₁₉ in the general formula (3), and these groups may be bonded to each other via a linking group such as a single bond, a substituted or unsubstituted methylene group, an oxygen atom, a sulfur atom, and a monosubstituted amino group to form a ring.

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Examples of the "aromatic hydrocarbon group", "aromatic heterocyclic group", or "fused polycyclic aromatic group" in the "disubstituted amino group substituted with a group selected from the group consisting of an aromatic hydrocarbon group, an aromatic heterocyclic group, and a fused polycyclic aromatic group" represented by R₂₆ to R₂₉ in the general formula (5) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by R₈ to R₁₉ in the general formula (3).

Further, these groups may each have a substituent group. Examples of the substituent group include the similar ones as described for the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3), and aspects similar to those of the "substituent group" in the "linear or branched alkyl group having 1 to 6 carbon atoms having a substituent group", "cycloalkyl group having 5 to 10 carbon atoms having a substituent group", or "linear or branched alkenyl group having 2 to 6 carbon atoms having a substituent group" represented by R₈ to R₁₉ in the general formula (3) can be taken.

Regarding the "disubstituted amino group substituted with a group selected from the group consisting of an aromatic hydrocarbon group, an aromatic heterocyclic group, and a fused polycyclic aromatic group" represented by R₂₆ to R₂₉ in the general formula (5), these groups (R₂₆ to R₂₉) may be bonded to each other via the "aromatic hydrocarbon group", "aromatic heterocyclic group", or "fused polycyclic aromatic group" of these groups (R₂₆ to R₂₉) and via a single bond, a substituted or unsubstituted methylene group, an oxygen atom, or a sulfur atom to form a ring. Alternatively, these groups (R₂₆ to R₂₉) and the benzene ring to which these groups (R₂₆ to R₂₉) are directly bonded may be bonded to each other via the "aromatic hydrocarbon group", "aromatic heterocyclic group", or "fused polycyclic aromatic group" of these groups (R₂₆ to R₂₉) and via a linking group such as a substituted or unsubstituted methylene group, an oxygen atom, a sulfur atom, and a monosubstituted amino group to form a ring.

In the general formula (1), as Ar₁, a "substituted or unsubstituted aromatic hydrocarbon group" or a "substituted or unsubstituted fused polycyclic aromatic group" is favorable, a substituted or unsubstituted phenyl group, a biphenylyl group, a terphenylyl group, a naphthyl group, a phenanthrenyl group, an anthracenyl group, a fluorenyl group, a carbazolyl group, an indolyl group, a dibenzofuranyl group, or a dibenzothienyl group is more favorable, and a substituted or unsubstituted phenyl group is particularly favorable.

In the general formula (1), as Ar₂, a "substituted or unsubstituted aromatic hydrocarbon group" or a "substituted or unsubstituted fused polycyclic aromatic group" is favorable, a substituted or unsubstituted phenyl group, a biphenylyl group, a terphenylyl group, a naphthyl group, a phenanthrenyl group, an anthracenyl group, or a fluorenyl group is more favorable, and a substituted or unsubstituted phenyl group is particularly favorable.

In the general formula (1), as Ar₃, a "substituted or unsubstituted aromatic hydrocarbon group" or a "substituted or unsubstituted fused polycyclic aromatic group" is favorable, a substituted or unsubstituted phenyl group, a biphenylyl group, a terphenylyl group, a naphthyl group, or a fluorenyl group is favorable, and a substituted or unsubstituted phenyl group is particularly favorable.

Further, of the compounds represented by the general formula (1), the compound represented by the general formula (1a) is more favorable.

As Ar₄ in the general formula (2), a phenyl group, a biphenylyl group, a naphthyl group, an anthracenyl group, an acenaphthenyl group, a phenanthrenyl group, a fluorenyl group, an indenyl group, a pyrenyl group, a perylenyl group, a fluoranthenyl group, or a triphenylenyl group is favorable, and a phenyl group, a biphenylyl group, a naphthyl group, an anthracenyl group, a phenanthrenyl group, a pyrenyl group, or a fluoranthenyl group, or a triphenylenyl group is more favorable. Here, a phenyl group favorably has a substituted or unsubstituted fused polycyclic aromatic group as a substituent group, and more favorably has a substituent group selected from the group consisting of a naphthyl group, an anthracenyl group, a phenanthrenyl group, a pyrenyl group, a fluoranthenyl group, and a triphenylenyl group.

As Ar₅ in the general formula (2), a phenyl group having a substituent group is favorable. As the substituent group in this case, an aromatic hydrocarbon group such as a phenyl group, a biphenylyl group, and a terphenyl group, or a fused polycyclic aromatic group such as a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group is favorable, and a phenyl group, a naphthyl group, an anthracenyl group, a phenanthrenyl group, a pyrenyl group, a fluoranthenyl group, or a triphenylenyl group is more favorable.

As Ar₆ in the general formula (2), a phenyl group having a substituent group is favorable. As the substituent group in this case, an aromatic hydrocarbon group such as a phenyl group, a biphenylyl group, and a terphenyl group, or a fused polycyclic aromatic group such as a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group is favorable, and a phenyl group, a naphthyl group, an anthracenyl group, a phenanthrenyl group, a pyrenyl group, a fluoranthenyl group, or a triphenylenyl group is more favorable.

In the general formula (2), Ar₅ and Ar₆ may be the same group. However, there is a possibility that crystallization easily occurs due to the improved symmetry of a whole molecule and it is favorable that Ar₅ and Ar₆ represent different groups from the viewpoint of the stability in a thin-film state. There is no case that both Ar₅ and Ar₆ are hydrogen atoms.

In the general formula (2), it is favorable that one of Ar₅ and Ar₆ represents a hydrogen atom.

As Ar₇ in the general formula (2), a triazinyl group, a pyridyl group, a pyrimidinyl group, a quinolyl group, an isoquinolyl group, an indolyl group, a quinoxalinyl group, a benzimidazolyl group, a naphthyridinyl group, a phenanthrolinyl group, or an acridinyl group is favorable, and a pyridyl group, a pyrimidinyl group, a quinolyl group, an isoquinolyl group, an indolyl group, a quinoxalinyl group, a benzimidazolyl group, a phenanthrolinyl group, or an acridinyl group is more favorable.

As R₈ to R₁₉ in the general formula (3), a deuterium atom, a "linear or branched alkyl group having 1 to 6 carbon atoms which may have a substituent group", a "linear or branched alkenyl group having 2 to 6 carbon atoms which may have a substituent group", a "substituted or unsubstituted aromatic hydrocarbon group", or a "substituted or unsubstituted fused polycyclic aromatic group" is favorable, and a deuterium atom, a phenyl group, a biphenylyl group, a naphthyl group, or a vinyl group is more favorable, and it is also favorable that these groups are bonded to each other via a single bond to form a fused aromatic ring. In particular, a deuterium atom, a phenyl group, or a biphenylyl group is favorable.

As R₈ to R₁₉ in the general formula (3), an integer of 0 to 3 is favorable and an integer of 0 to 2 is more favorable.

As L₁ to L₃ in the general formula (3), a divalent group represented by the structural formula (B) or (D) or a single bond is favorable, and a divalent group represented by the structural formula (B) or a single bond is more favorable.

As n1 in the structural formula (B) of the general formula (3), 1 or 2 is favorable and 1 is more favorable.

As R₂₀ to R₂₅ in the general formula (4), a deuterium atom, a "linear or branched alkyl group having 1 to 6 carbon atoms which may have a substituent group", a "linear or branched alkenyl group having 2 to 6 carbon atoms which may have a substituent group", a "substituted or unsubstituted aromatic hydrocarbon group", or a "substituted or unsubstituted fused polycyclic aromatic group" is favorable, and a deuterium atom, a phenyl group, a biphenylyl group, a naphthyl group, or a vinyl group is more favorable, and it is also favorable that these groups are bonded to each other via a single bond to form a fused aromatic ring. In particular, a deuterium atom, a phenyl group, or a biphenylyl group is favorable.

As R₂₀ to R₂₅ in the general formula (4), an integer of 0 to 3 is favorable and an integer of 0 to 2 is more favorable.

As L₄ in the general formula (4), a divalent group represented by the structural formula (B), (D), or (G) or a single bond is favorable, and a divalent group represented by the structural formula (D) or (G) or a single bond is more favorable.

As n1 in the structural formula (B) of the general formula (4), 1 or 2 is favorable.

As A₁ in the general formula (5), a "divalent group of a substituted or unsubstituted aromatic hydrocarbon" or a single bond is favorable, a divalent group formed by removing two hydrogen atoms from benzene, biphenyl, or naphthalene, or a single bond is more favorable, and a single bond is particularly favorable.

As Ar₉ and Ar₁₀ in the general formula (5), a phenyl group, a biphenylyl group, a naphthyl group, a fluorenyl group, an indenyl group, a pyridyl group, a dibenzofuranyl group, or a pyridobenzofuranyl group is favorable.

Ar₉ and Ar₁₀ in the general formula (5) may be bonded to each other directly or via a substituent group of the groups and via a single bond, a substituted or unsubstituted methylene group, an oxygen atom, or a sulfur atom to form a ring.

It is favorable that at least one of R₂₆ to R₂₉ in the general formula (5) represents a "disubstituted amino group substituted with a group selected from the group consisting of an aromatic hydrocarbon group, an aromatic heterocyclic group, and a fused polycyclic aromatic group" is favorable. As the "aromatic hydrocarbon group", "aromatic heterocyclic group", or "fused polycyclic aromatic group" in this case, a phenyl group, a biphenylyl group, a naphthyl group, a fluorenyl group, an indenyl group, a pyridyl group, a dibenzofuranyl group, or a pyridobenzofuranyl group is favorable.

In the general formula (5), an aspect in which adjacent two or all of R₂₆ to R₂₉ represent vinyl groups and adjacent two vinyl groups are bonded to each other via a single bond to form a ring, i.e., an aspect in which R₂₆ to R₂₉ and the benzene ring to which R₂₆ to R₂₉ are bonded form a naphthalene ring or a phenanthrene ring is also favorable.

In the general formula (5), an aspect in which one of R₂₆ to R₂₉ represents an "aromatic hydrocarbon group" and the "aromatic hydrocarbon group" and the benzene ring to which R₂₆ to R₂₉ are bonded are bonded to each other via a substituted or unsubstituted methylene group, an oxygen atom, or a sulfur atom to form a ring is favorable. An aspect in which the "aromatic hydrocarbon group" in this case represents a phenyl group and the "aromatic hydrocarbon group" and the benzene ring to which R₂₆ to R₂₉ are bonded are bonded to each other via an oxygen atom or a sulfur atom to form a ring, i.e., an aspect in which the "aromatic hydrocarbon group" and the benzene ring to which R₂₆ to R₂₉ are bonded are bonded to each other to form a dibenzofuran ring or a dibenzothiophene ring is particularly favorable.

In the general formula (5), an aspect in which one of R₃₀ to R₃₂ represents an "aromatic hydrocarbon group" and the "aromatic hydrocarbon group" and the benzene ring to which R₃₀ to R₃₂ are bonded are bonded to each other to form a ring via a substituted or unsubstituted methylene group, an oxygen atom, or a sulfur atom is favorable. An aspect in which the "aromatic hydrocarbon group" in this case represents a phenyl group and the phenyl group and the benzene ring to which R₃₀ to R₃₂ are bonded are bonded to each other via an oxygen atom or a sulfur atom to form a ring, i.e., an aspect in which a dibenzofuran ring or a dibenzothiophene ring is formed from the phenyl group and the benzene ring to which R₃₀ to R₃₂ are bonded is particularly favorable.

As R₃₃ and R₃₄ in the general formula (5), a "substituted or unsubstituted aromatic hydrocarbon group", a "substituted or unsubstituted aromatic heterocyclic group containing oxygen", or a "substituted or unsubstituted fused polycyclic aromatic group" is favorable, a phenyl group, a naphthyl group, a phenanthrenyl group, a pyridyl group, a quinolyl group, an isoquinolyl group, or a dibenzofuranyl group is more favorable, and a phenyl group is particularly favorable.

Then, an aspect in which R₃₃ and R₃₄ are bonded to each other via a linking group such as a single bond, a substituted or unsubstituted methylene group, an oxygen atom, a sulfur atom, and a monosubstituted amino group to form a ring is favorable, and an aspect in which R₃₃ and R₃₄ are bonded to each other via a single bond to form a ring is particularly favorable.

### Advantageous Effects of Invention

The arylamine compound represented by the general formula (1), which is suitably used for the organic EL device according to the present invention, can be used as a constituent material of a hole transport layer of the organic EL device. The arylamine compound represented by the general formula (1) has properties of (1) having a high hole injection property, (2) having a high mobility of holes, (3) having excellent electron blocking capability, (4) having high stability in a thin-film state, and (5) having an excellent heat resistance.

Since an arylamine compound that has a higher mobility of holes, excellent electron blocking performance, an excellent amorphous property, and higher stability in a thin-film state as compared with the existing hole transport material is used for the organic EL device according to the present invention, it is possible to realize an organic EL device having high efficiency, a low drive voltage, and a long lifetime.

Further, in the present invention, by forming a hole transport layer to have a two-layer structure of a first hole transport layer and a second hole transport layer and forming a second hole transport layer of the arylamine compound represented by the general formula (1), which is located on the side adjacent to the light-emitting layer, it is possible to make the most of the electron blocking performance of the arylamine compound and realize an organic EL device having high efficiency and a long lifetime.

### Brief Description of Drawings

[Fig. 1] Fig. 1 is a diagram showing structural formulae of compounds 1-1 to 1-18 as the arylamine compound represented by the general formula (1) .
[Fig. 2] Fig. 2 is a diagram showing structural formulae of compounds 1-19 to 1-33 as the arylamine compound represented by the general formula (1).
[Fig. 3] Fig. 3 is a diagram showing structural formulae of compounds 1-34 to 1-48 as the arylamine compound represented by the general formula (1).
[Fig. 4] Fig. 4 is a diagram showing structural formulae of compounds 1-49 to 1-63 as the arylamine compound represented by the general formula (1).
[Fig. 5] Fig. 5 is a diagram showing structural formulae of compounds 1-64 to 1-78 as the arylamine compound represented by the general formula (1).
[Fig. 6] Fig. 6 is a diagram showing structural formulae of compounds 1-79 to 1-93 as the arylamine compound represented by the general formula (1).
[Fig. 7] Fig. 7 is a diagram showing structural formulae of compounds 1-94 to 1-105 as the arylamine compound represented by the general formula (1).
[Fig. 8] Fig. 8 is a diagram showing structural formulae of compounds 2-1 to 2-15 as the compound having a pyrimidine ring structure represented by the general formula (2).
[Fig. 9] Fig. 9 is a diagram showing structural formulae of compounds 2-16 to 2-30 as the compound having a pyrimidine ring structure represented by the general formula (2).
[Fig. 10] Fig. 10 is a diagram showing structural formulae of compounds 2-31 to 2-45 as the compound having a pyrimidine ring structure represented by the general formula (2).
[Fig. 11] Fig. 11 is a diagram showing structural formulae of compounds 2-46 to 2-60 as the compound having a pyrimidine ring structure represented by the general formula (2).
[Fig. 12] Fig. 12 is a diagram showing structural formulae of compounds 2-61 to 2-75 as the compound having a pyrimidine ring structure represented by the general formula (2).
[Fig. 13] Fig. 13 is a diagram showing structural formulae of compounds 2-76 to 2-87 as the compound having a pyrimidine ring structure represented by the general formula (2).
[Fig. 14] Fig. 14 is a diagram showing structural formulae of compounds 2-88 to 2-99 as the compound having a pyrimidine ring structure represented by the general formula (2).
[Fig. 15] Fig. 15 is a diagram showing structural formulae of compounds 2-100 to 2-111 as the compound having a pyrimidine ring structure represented by the general formula (2).
[Fig. 16] Fig. 16 is a diagram showing structural formulae of compounds 2-112 to 2-123 as the compound having a pyrimidine ring structure represented by the general formula (2).
[Fig. 17] Fig. 17 is a diagram showing structural formulae of compounds 2-124 to 2-126 as the compound having a pyrimidine ring structure represented by the general formula (2).
[Fig. 18] Fig. 18 is a diagram showing structural formulae of compounds 3-1 to 3-8 as the triphenylamine derivative represented by the general formula (3).
[Fig. 19] Fig. 19 is a diagram showing structural formulae of compounds 3-9 to 3-17 as the triphenylamine derivative represented by the general formula (3).
[Fig. 20] Fig. 20 is a diagram showing structural formulae of compounds 4-1 to 4-15 as the triphenylamine derivative represented by the general formula (4).

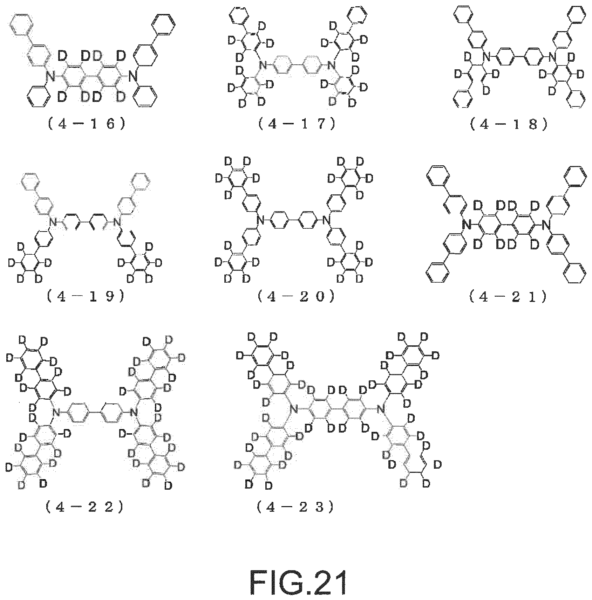

[Fig. 21] Fig. 21 is a diagram showing structural formulae of compounds 4-16 to 4-23 as the triphenylamine derivative represented by the general formula (4).
[Fig. 22] Fig. 22 is a diagram showing structural formulae of compounds 5-1 to 5-6 as the amine derivative having a fused ring structure represented by the general formula (5).
[Fig. 23] Fig. 23 is a diagram showing structural formulae of compounds 5-7 to 5-21 as the amine derivative having a fused ring structure represented by the general formula (5).
[Fig. 24] Fig. 24 is a diagram showing configurations of organic EL devices according to Examples 11 to 18 and Comparative Examples 1 and 2. Mode(s) for Carrying Out the Invention

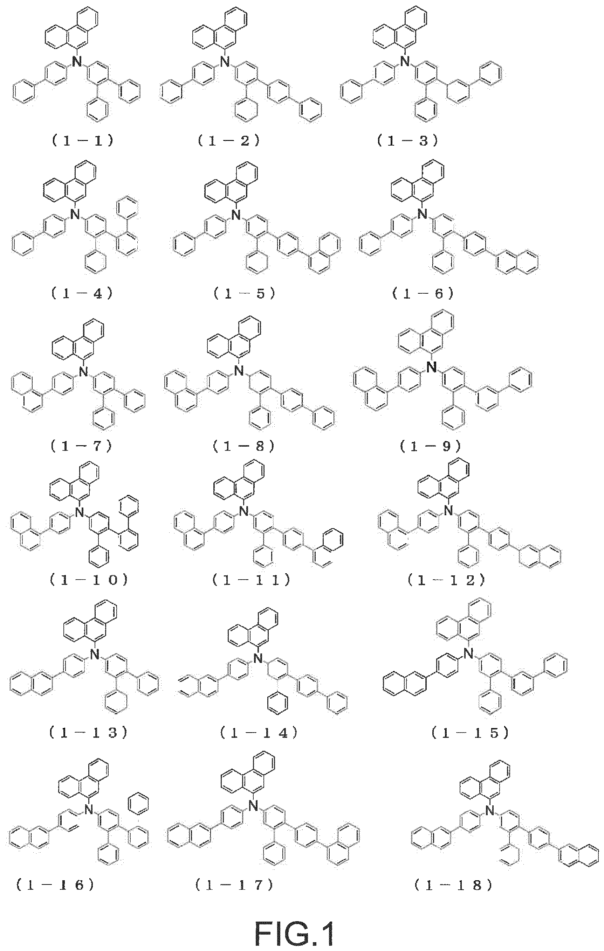

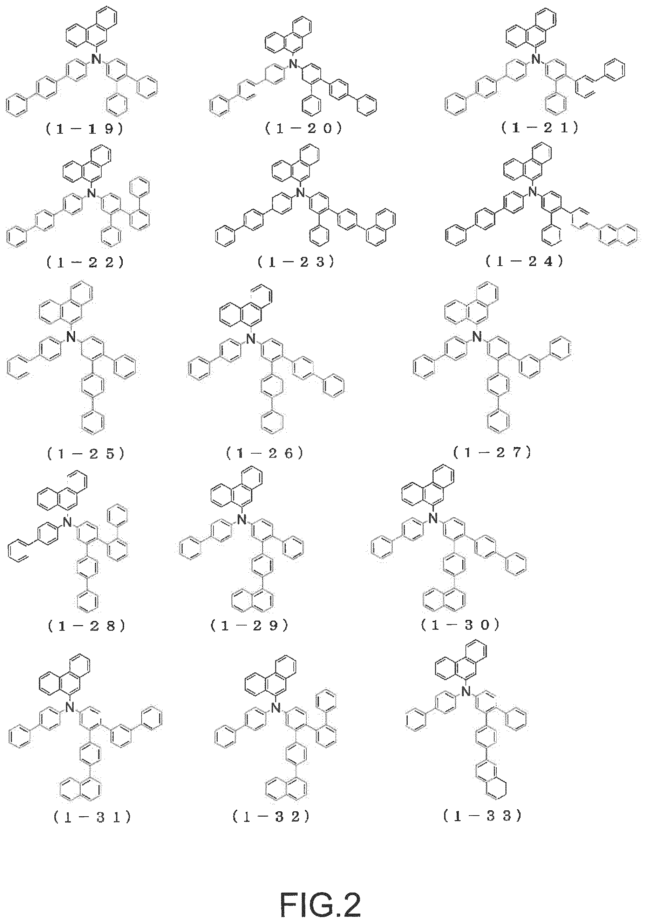

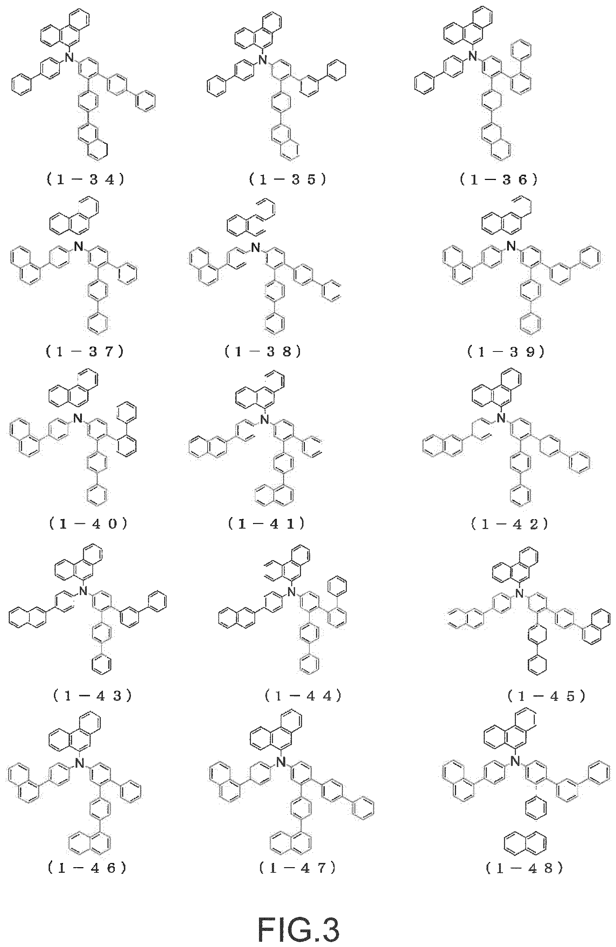

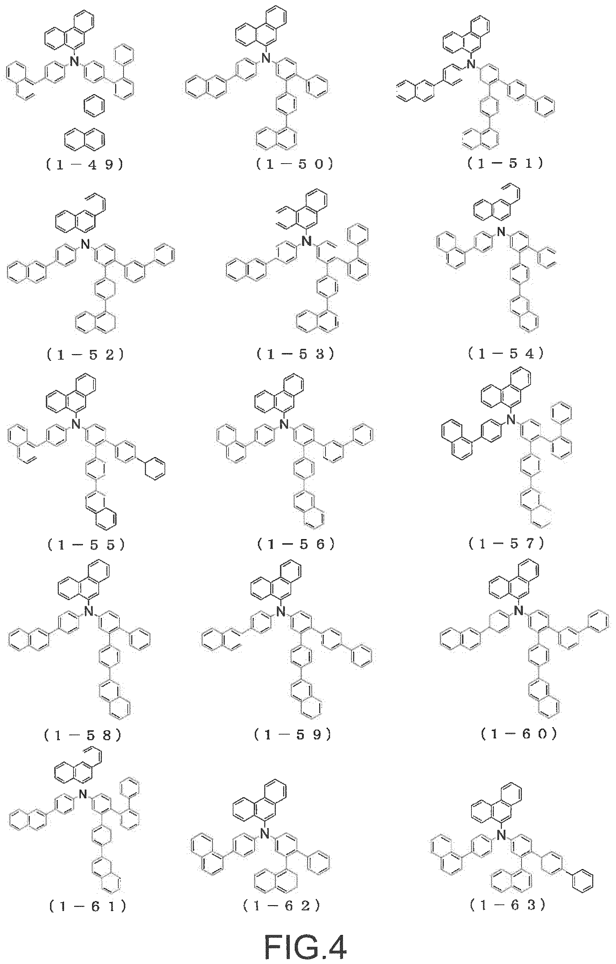

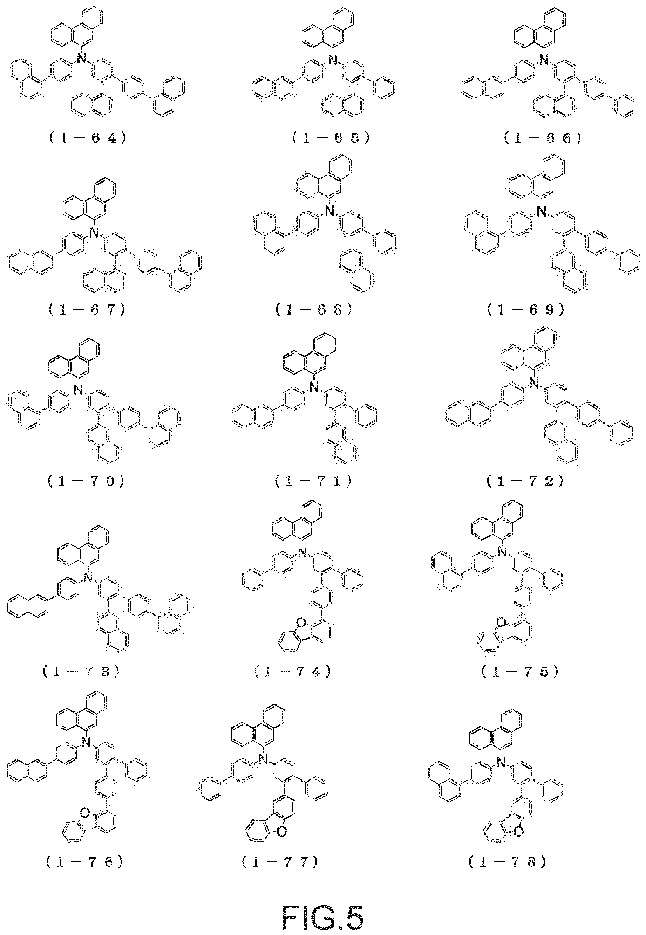

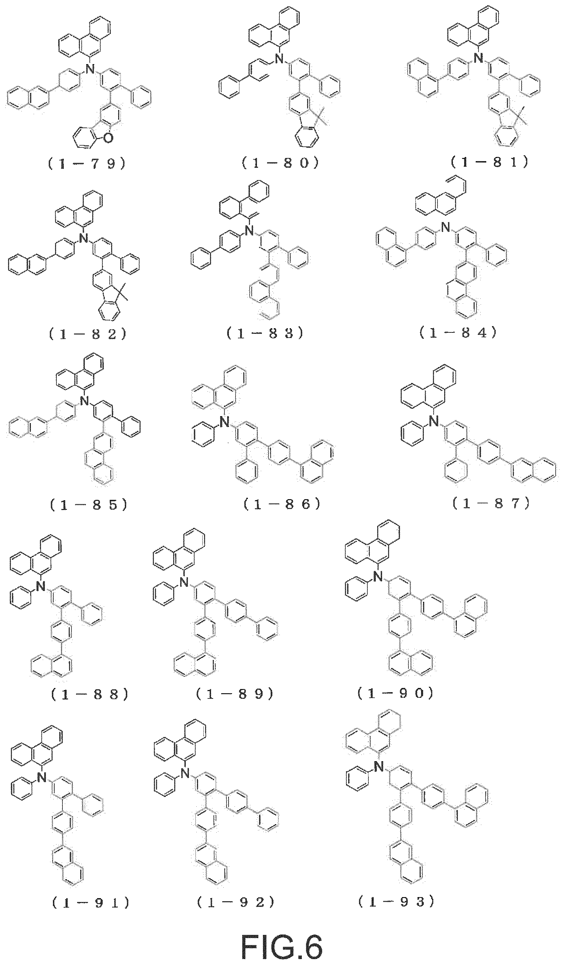

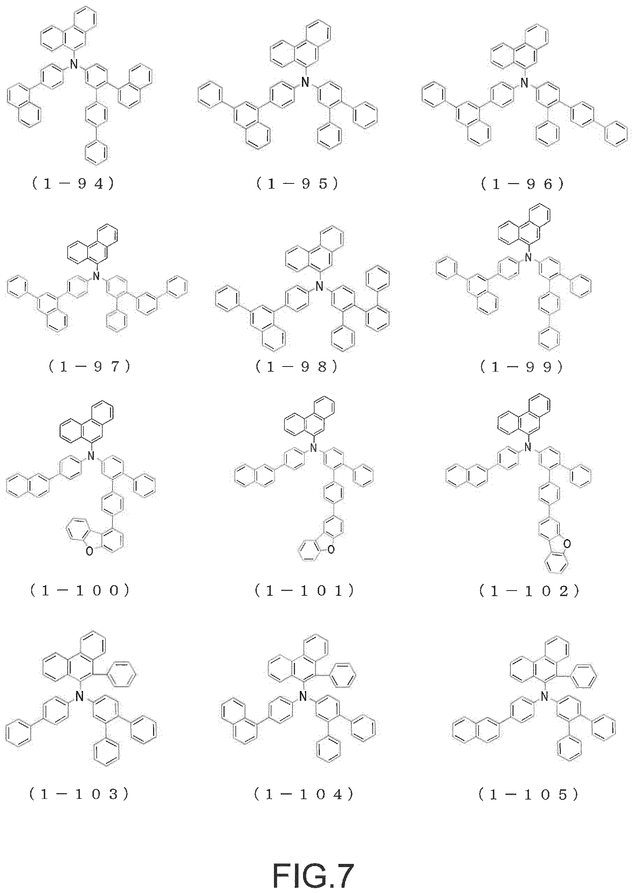

Specific examples of favorable compounds of the arylamine compound represented by the general formula (1), which is suitably used for the organic EL device according to the present invention, are shown in Fig. 1 to Fig. 7, but the present invention is not limited to these compounds.

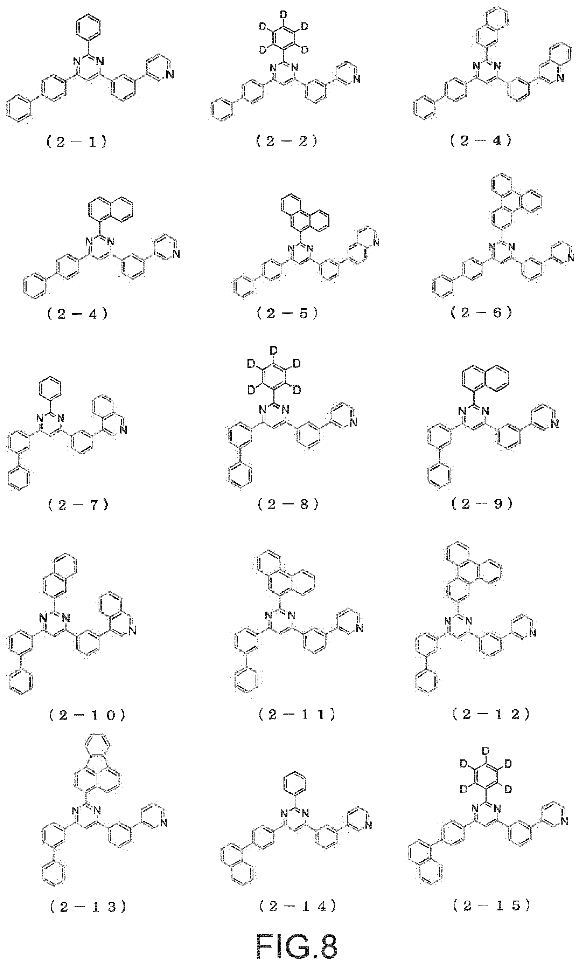

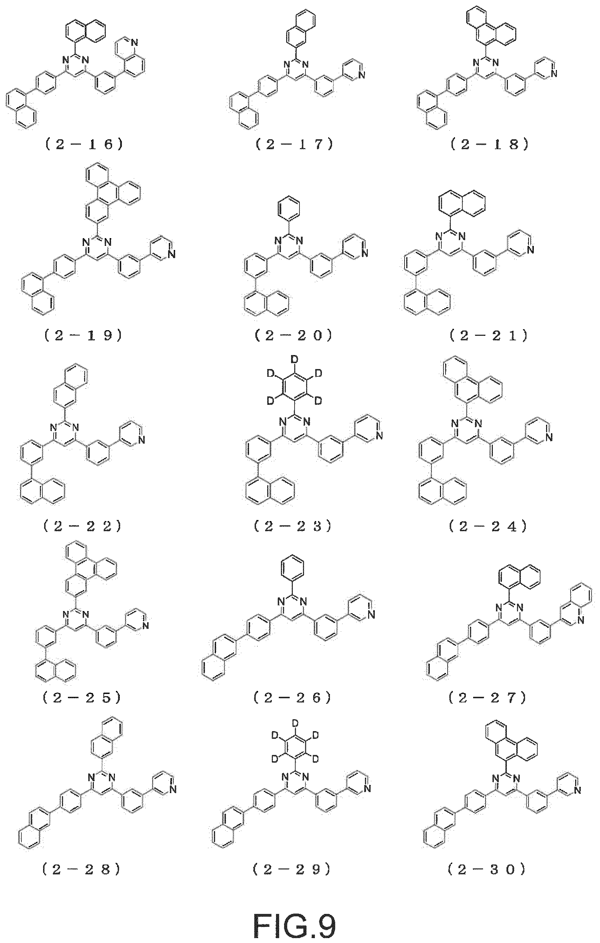

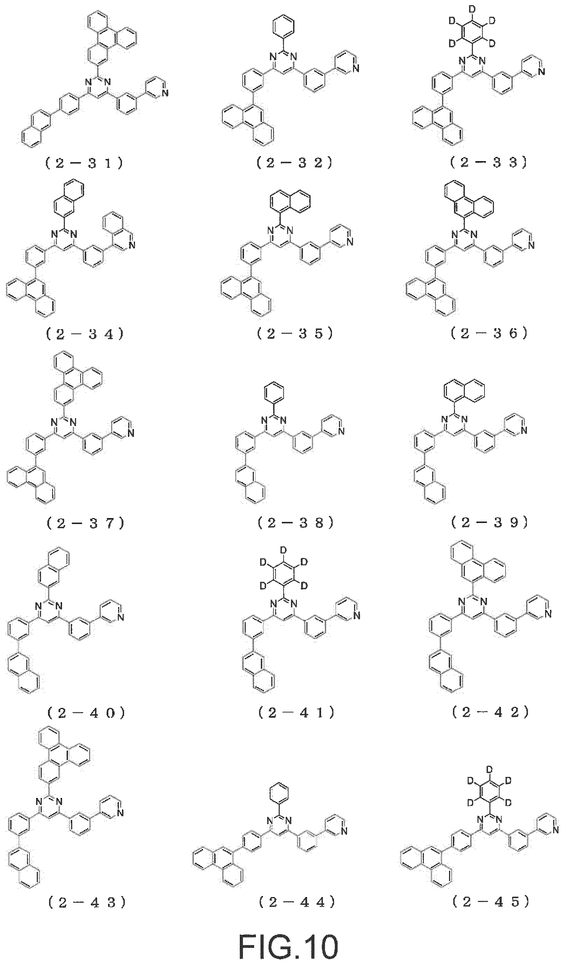

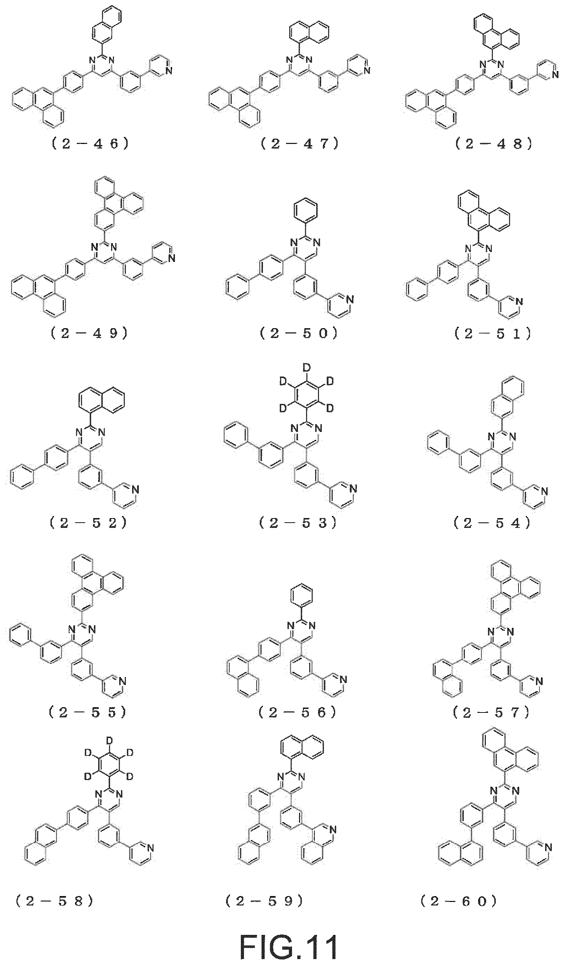

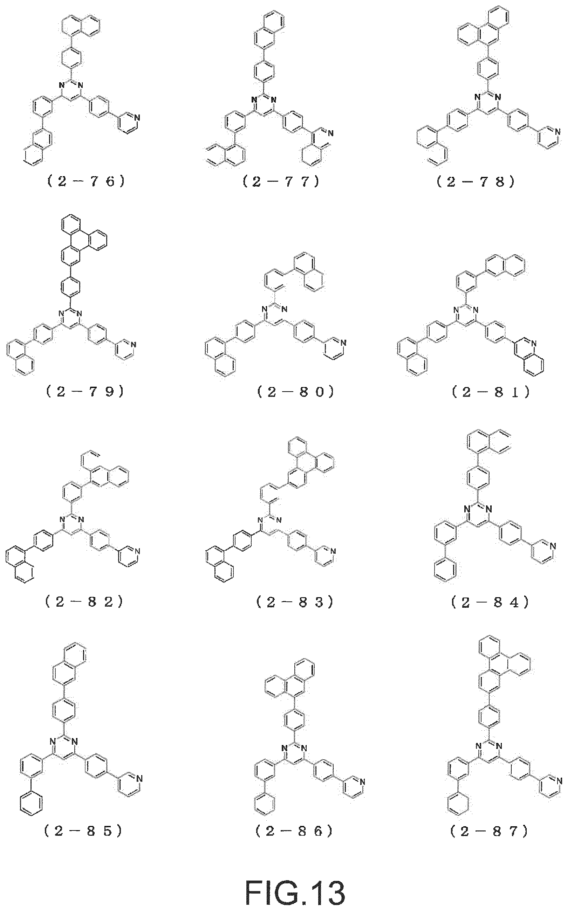

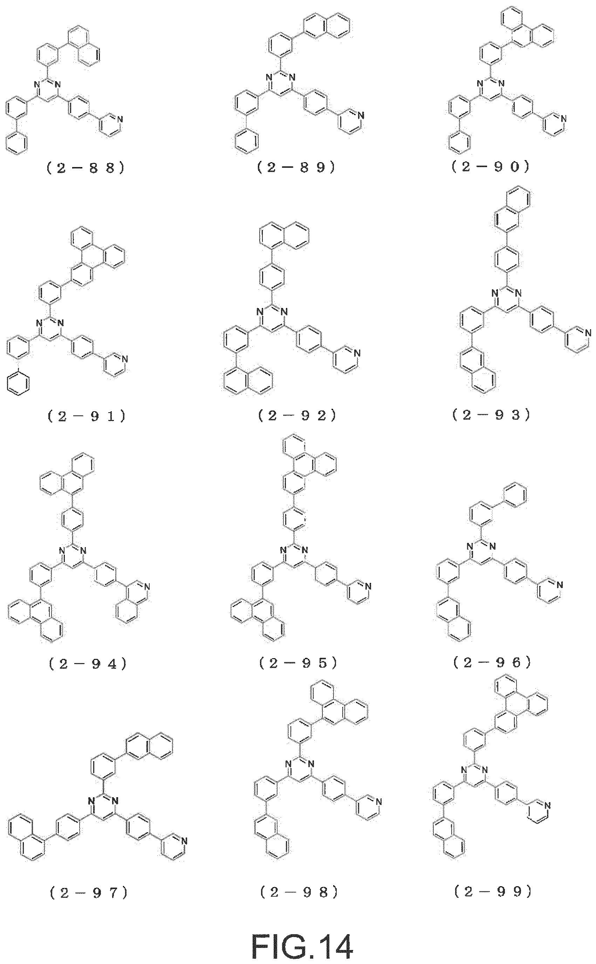

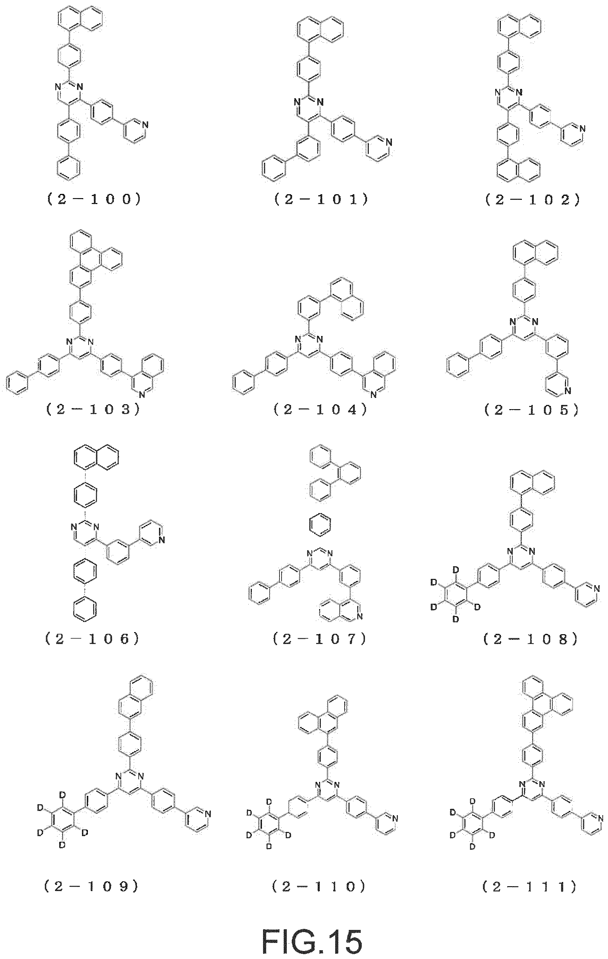

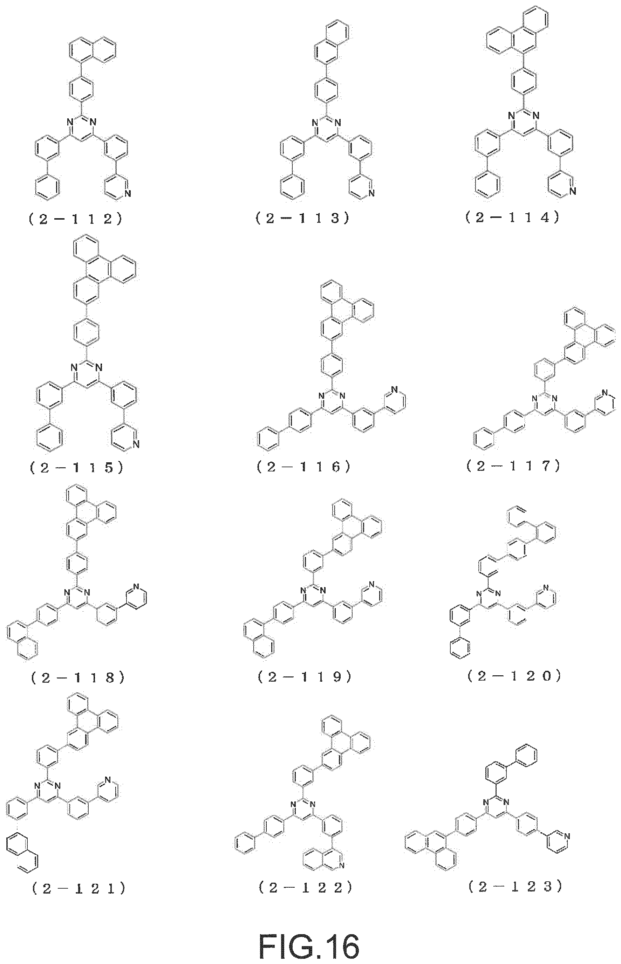

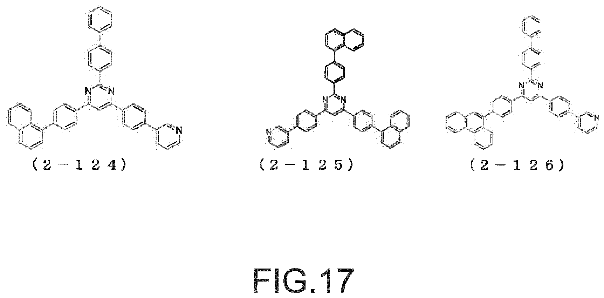

Specific examples of favorable compounds of the compound having a pyrimidine ring structure represented by the general formula (2), which is suitably used for the organic EL device according to the present invention, are shown in Fig. 8 to Fig. 17, but the present invention is not limited to these compounds.

Note that the above-mentioned compound having a pyrimidine ring structure can be synthesized in accordance with a method known per se can be synthesized in accordance with a method known per se (see, for example, Patent Literature 6).

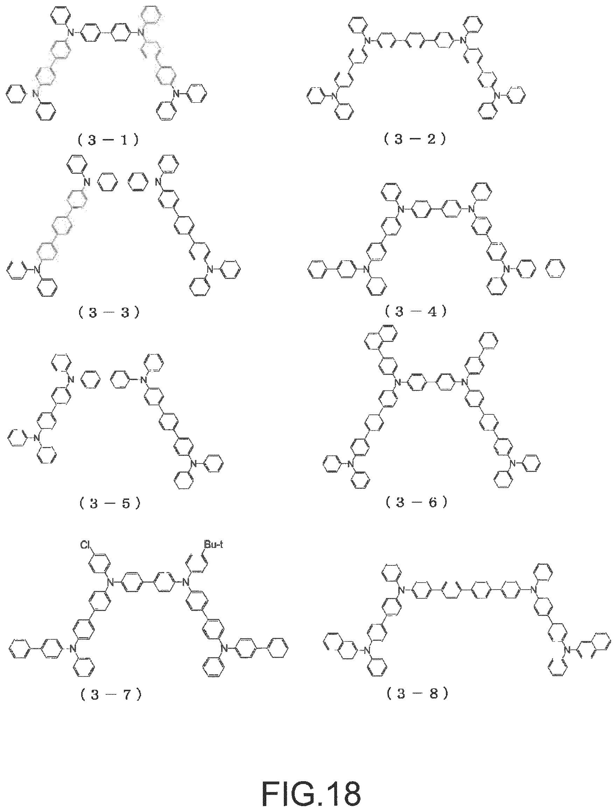

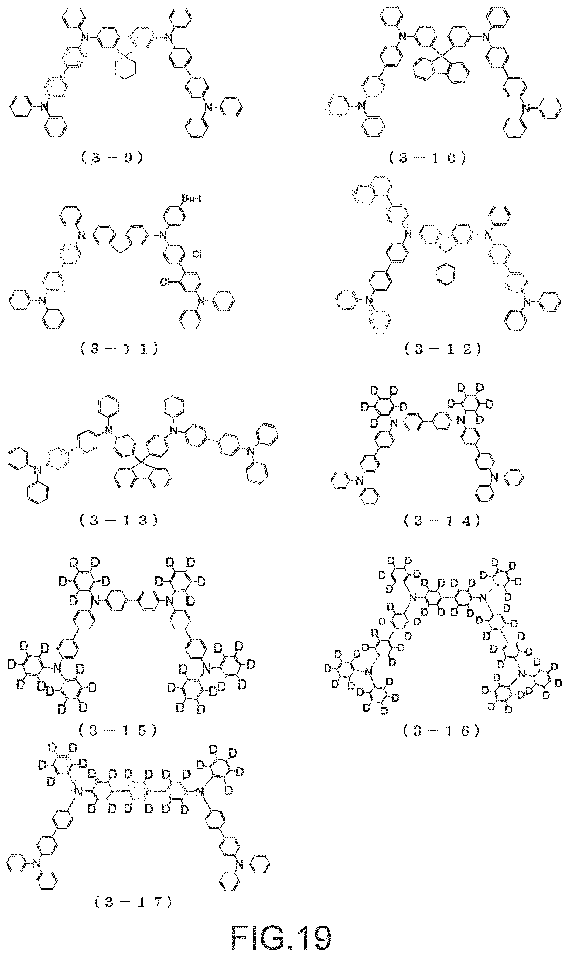

Specific examples of favorable compounds of the triphenylamine derivative represented by the general formula (3), which is suitably used for the organic EL device according to the present invention, are shown in Fig. 18 and Fig. 19, but the present invention is not limited to these compounds.

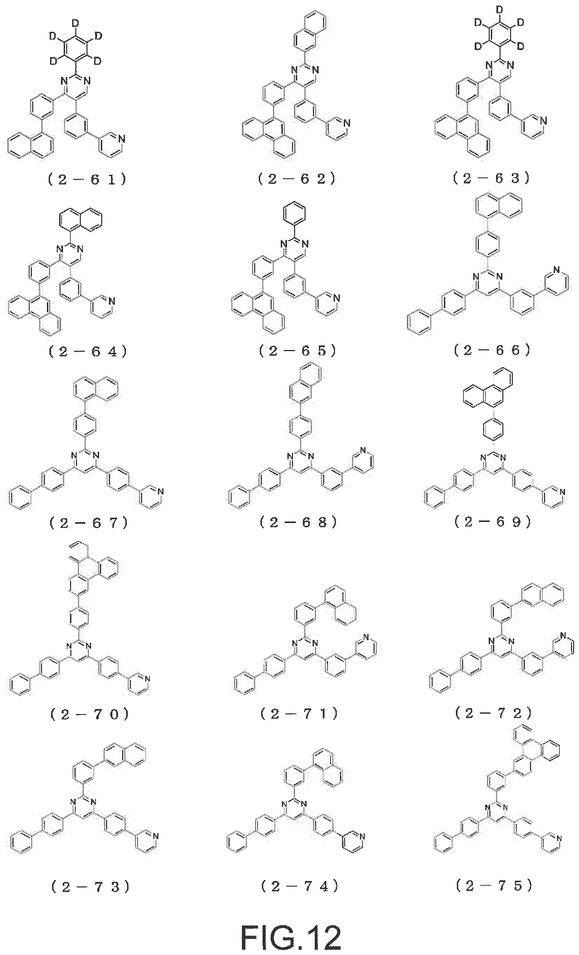

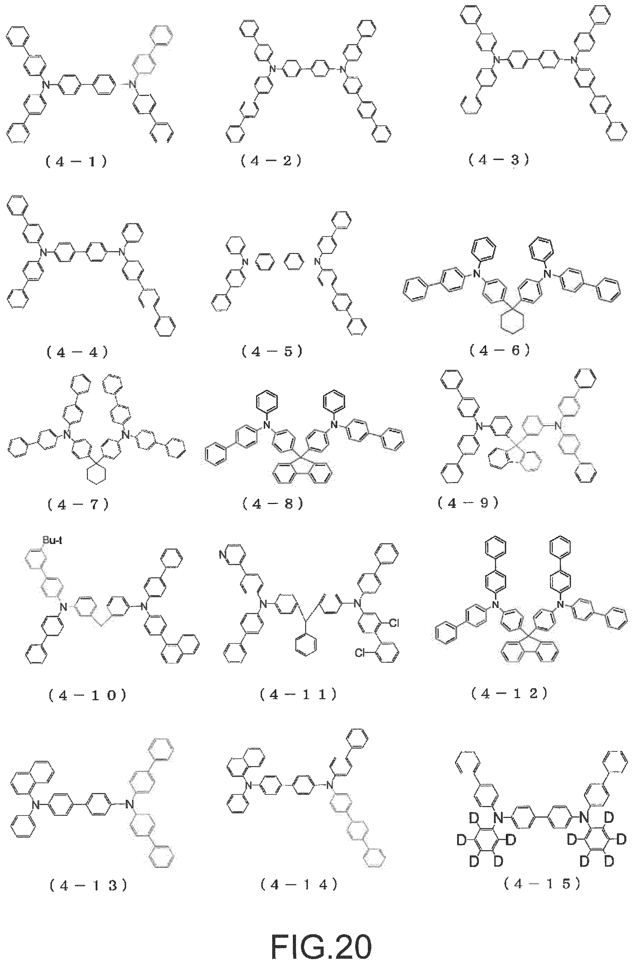

Specific examples of favorable compounds of the triphenylamine derivative represented by the general formula (4), which is suitably used for the organic EL device according to the present invention, are shown in Fig. 20 and Fig. 12, but the present invention is not limited to these compounds.

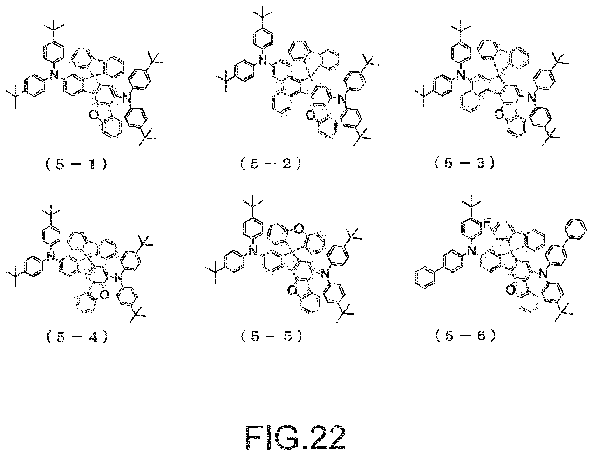

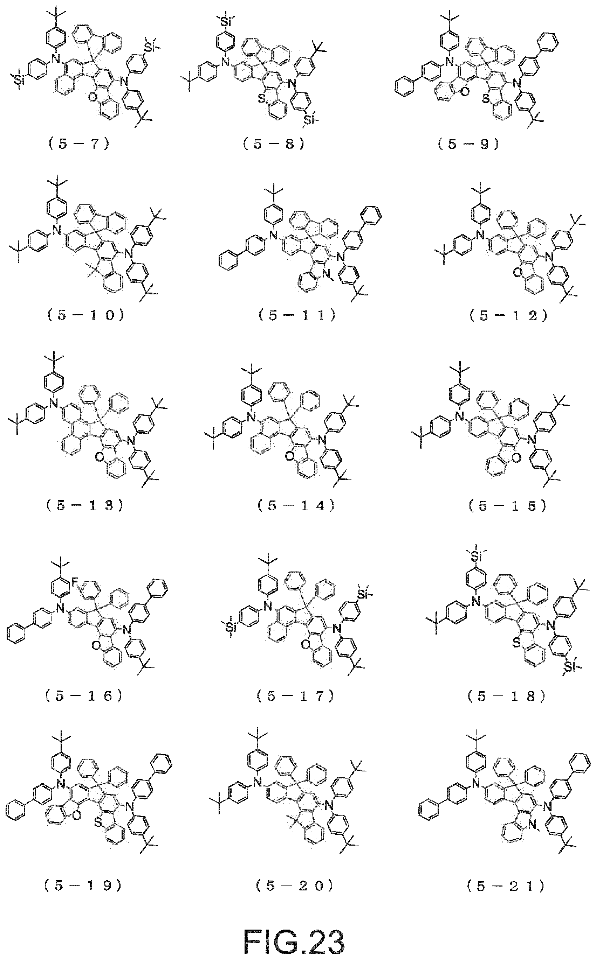

Specific examples of favorable compounds of the amine derivative having a fused ring structure represented by the general formula (5), which is suitably used for the organic EL device according to the present invention, are shown in Fig. 22 and Fig. 23, but the present invention is not limited to these compounds.

Note that the above-mentioned amine derivative having a fused ring structure can be synthesized in accordance with a method known per se (see, for example, Patent Literature 7).

Purification for the arylamine compound represented by the general formula (1) was carried out by purification by column chromatography, adsorption purification with silica gel, activated carbon, activated clay, or the like, recrystallization with a solvent, a crystallization method, a sublimation purification method, or the like. Identification of the compounds was performed by NMR analysis. As physical property values, a melting point, a glass transition point (Tg), and a work function were measured. The melting point is an index of a vapor deposition property. The glass transition point (Tg) is an index of stability in a thin film state. The work function is an index of a hole transport property and a hole blocking property.

In addition, regarding the compound used for the organic EL device according to the present invention, those purified by purification by column chromatography, adsorption purification with silica gel, activated carbon, activated clay, or the like, recrystallization with a solvent, a crystallization method, a sublimation purification method, or the like, and finally purified by a sublimation purification method were used.

The melting point and the glass transition point (Tg) were measured with a powder using a high sensitivity differential scanning calorimeter (DSC3100SA manufactured by Bruker AXS GmbH).

The work function was obtained by preparing a thin film of 100 nm on an ITO substrate and using an ionization potential measuring apparatus (PYS-202 manufactured by Sumitomo Heavy Industries, Ltd.).

Examples of the structure of the organic EL device according to the present invention include those including an anode, a hole transport layer, a light-emitting layer, an electron transport layer, and a cathode in the stated order on a substrate, those including an anode, a hole transport layer, a light-emitting layer, an electron transport layer, and a cathode, those including a hole injection layer between an anode and a hole transport layer, those including a hole blocking layer between a light-emitting layer and an electron transport layer, and those including an electron injection layer between an electron transport layer and a cathode. In the multilayer structures, several organic layers can be omitted or combined. For example, the hole injection layer and the hole transport layer may be combined, or the electron injection layer and the electron transport layer may be combined. Further, two or more organic layers having the same function can be stacked. For example, two hole transport layers may be stacked, two light-emitting layers may be stacked, or two electron transport layers may be stacked. As the structure of the organic EL device according to the present invention, it is favorable that the hole transport layer has a two-layer structure of the first hole transport layer and the second hole transport layer. The second hole transport layer in this case is adjacent to the light-emitting layer and has a function as an electron blocking layer.

For the anode of the organic EL device according to the present invention, an electrode material having a large work function, such as ITO and gold, is used. As the hole injection layer of the organic EL device according to the present invention, a material such as a starburst triphenylamine derivative and various triphenylamine tetramers; a porphyrin compound typified by copper phthalocyanine; an acceptor heterocyclic compound such as hexacyanoazatriphenylene, a coating type polymer material, or the like can be used. These materials can be formed into a thin film by a known method such as a spin coat method and an ink jet method in addition to a vapor deposition method.

The arylamine compound represented by the general formula (1) is used for the hole transport layer of the organic EL device according to the present invention. As the hole transport material that can be mixed with or used together with the arylamine compound represented by the general formula (1), a compound such as various triphenylamine derivatives can be used in addition to a benzidine derivative such as N,N'-diphenyl-N,N'-di(m-tolyl)benzidine (TPD), N,N'-diphenyl-N,N'-di(α-naphthyl)benzidine (NPD), and N,N,N',N'- tetrabiphenylylbenzidine, 1,1-bis[4-(di-4-tolylamino)phenyl]cyclohexane (TAPC), and the triphenylamine derivative represented by the general formula (3) or (4). These materials may be deposited alone. However, one of the materials may be mixed with another material and used as a single deposited layer. Further, a stacked structure of layers deposited alone, which are each formed of one of the plurality of materials, layers mixed and deposited, which are each formed of the plurality of materials, or at least one layer deposited alone, which is formed of one of the plurality of materials, and at least one layer mixed and deposited, which is formed of the plurality of materials, may be achieved. These materials can be formed into a thin film by a known method such as a spin coat method and an ink jet method in addition to a vapor deposition method.

Further, in the hole injection layer or the hole transport layer, those obtained by P-doping the material typically used for the respective layers with trisbromophenylaminehexachloroantimony or a radialene derivative (see, for example, Patent Literature 8), a polymer compound having, as a partial structure, the structure of a benzidine derivative such as TPD, or the like can be used.

In the case where the hole transport layer of the organic EL device according to the present invention has a two-layer structure of the first hole transport layer and the second hole transport layer, the arylamine compound represented by the general formula (1) is used for the second hole transport layer adjacent to the light-emitting layer. Examples of the hole transport material that can be mixed with or used together with the arylamine compound represented by the general formula (1) include a carbazole derivative such as 4,4',4''-tri(N-carbazolyl) triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz), and a compound having an electron blocking effect, such as a compound having a triphenylsilyl group and a triarylamine structure typified by 9-[4-(carbazol-9-yl)phenyl]-9-[4-(triphenylsilyl)phenyl]-9H-fluorene.

These materials may be deposited alone. However, one of the materials may be mixed with another material and used as a single deposited layer. Further, a stacked structure of layers deposited alone, which are each formed of one of the plurality of materials, layers mixed and deposited, which are each formed of the plurality of materials, or at least one layer deposited alone, which is formed of one of the plurality of materials, and at least one layer mixed and deposited, which is formed of the plurality of materials, may be achieved. These materials can be formed into a thin film by a known method such as a spin coat method and an ink jet method in addition to a vapor deposition method.

For the light-emitting layer of the organic EL device according to the present invention, various metal complexes, an anthracene derivative, a bis-styrylbenzene derivative, a pyrene derivative, an oxazole derivative, a polyparaphenylene vinylene derivative, or the like can be used in addition to a metal complex of a quinolinol derivative including Alq₃. Further, the light-emitting layer may be formed of a host material and a dopant material, and an anthracene derivative is favorably used as the host material. In addition to the light-emitting material, a heterocyclic compound having an indole ring as a partial structure of a fused ring, a heterocyclic compound having a carbazole ring as a partial structure of a fused ring,
a carbazole derivative, a thiazole derivative, a benzimidazole derivative, a polydialkylfluorene derivative, or the like can be used. Further, as the dopant material, a pyrene derivative or the amine derivative having a fused ring structure represented by the general formula (5) is favorably used. In addition thereto, quinacridone, coumarin, rubrene, perylene, derivatives thereof, a benzopyran derivative, an indenophenanthrene derivative, a rhodamine derivative, an aminostyryl derivative, or the like can be used. These materials may be deposited alone. However, one of the materials may be mixed with another material and used as a single deposited layer. Further, a stacked structure of layers deposited alone, which are each formed of one of the plurality of materials, layers mixed and deposited, which are each formed of the plurality of materials, or at least one layer deposited alone, which is formed of one of the plurality of materials, and at least one layer mixed and deposited, which is formed of the plurality of materials, may be achieved.

Further, as the light-emitting material, a phosphorescent emitter can be used. As the phosphorescent emitter, a phosphorescent emitter of a metal complex such as iridium and platinum can be used. A green phosphorescent emitter such as Ir(ppy)₃, a blue phosphorescent emitter such as FIrpic and FIr6, a red phosphorescent emitter such as Btp₂Ir (acac), or the like is used. As the host material at this time, which is a host material having a hole injection/transport property, a carbazole derivative such as 4,4'-di (N-carbazolyl) biphenyl (CBP), TCTA, and mCP can be used. As the electron transport host material, p-bis(triphenylsilyl)benzene (UGH2), 2,2',2"-(1,3,5-phenylene)-tris(1-phenyl-1H-benzimidazole) (TPBI), or the like can be used, and an organic EL device having high performance can be prepared.

In order to avoid concentration quenching, it is favorable to dope the phosphorescent light-emitting material with the host material by co-deposition in the range of 1 to 30 weight percent with respect to the entire light-emitting layer.

Further, as the light-emitting material, a material that emits delayed fluorescence, such as a CDCB derivative including PIC-TRZ, CC2TA, PXZ-TRZ, and 4CzIPN can also be used (see, for example, Non-Patent Literature 3).

These materials can be formed into a thin film by a known method such as a spin coat method and an ink jet method in addition to a vapor deposition method.

For the hole blocking layer of the organic EL device according to the present invention, various rare earth complexes and a compound having a hole blocking effect, such as a triazole derivative, a triazine derivative, and an oxadiazole derivative, can be used in addition to a phenanthroline derivative such as bathocuproine (BCP) and a metal complex of a quinolinol derivative such as aluminum (III) bis(2-methyl-8-quinolinate)-4-phenylphenolate (hereinafter, abbreviated as BAlq). These materials may also serve as the material of the electron transport layer. These materials may be deposited alone. However, one of the materials may be mixed with another material and used as a single deposited layer. Further, a stacked structure of a combination of layers deposited alone; a combination of layers mixed and deposited; or a combination of a layer deposited alone and a layer mixed and deposited may be achieved. These materials can be formed into a thin film by a known method such as a spin coat method and an ink jet method in addition to a vapor deposition method.

For the electron transport layer of the organic EL device according to the present invention, the compound having a pyrimidine ring structure represented by the general formula (2) is favorably used. In addition thereto, a metal complex of a quinolinol derivative including Alq₃ and BAlq, various metal complexes, a triazole derivative, a triazine derivative, an oxadiazole derivative, a pyridine derivative, a pyrimidine derivative, a benzimidazole derivative, a thiadiazole derivative, an anthracene derivative, a carbodiimide derivative, a quinoxaline derivative, a pyridoindole derivative, a phenanthroline derivative, a silole derivative, or the like can be used. These materials may be deposited alone. However, one of the materials may be mixed with another material and used as a single deposited layer. Further, a stacked structure of a combination of layers deposited alone; a combination of layers mixed and deposited; or a combination of a layer deposited alone and a layer mixed and deposited; may be achieved. These materials can be formed into a thin film by a known method such as a spin coat method and an ink jet method in addition to a vapor deposition method.

For the electron injection layer of the organic EL device according to the present invention, an alkali metal salt such as lithium fluoride and cesium fluoride, an alkaline earth metal salt such as magnesium fluoride, a metal complex of a quinolinol derivative such as lithium quinolinol, a metal oxide such as aluminum oxide, a metal such as ytterbium (Yb), samarium (Sm), calcium (Ca), strontium (Sr), and cesium (Cs), or the like can be used. However, this can be omitted in the favorable selection of the electron transport layer and the cathode.

Further, in the electron injection layer or the electron transport layer, those obtained by N-doping the material typically used for the respective layers with a metal such as cesium can be used.

In the cathode of the organic EL device according to the present invention, an electrode material having a low work function, such as aluminum, or an alloy having a lower work function, such as a magnesium silver alloy, a magnesium indium alloy, and an aluminum magnesium alloy is used as the electrode material.

Hereinafter, the embodiment of the present invention will be specifically described by way of Examples. However, the present invention is not limited to the following Examples.

### (Example 1)

### <Synthesis of {4-(naphthalene-1-yl)phenyl}-(phenanthrene-9-yl)-{4"-(naphthalene-1-yl)-1,1':2',1"-terphenyl-5'-yl}amine (Compound 1-11)>

22.4 g of 4-(naphthalene-1-yl)phenylamine, 19.8 g of 3-bromobiphenyl, 12.2 g of t-butoxy sodium, and 230 mL of toluene were added to a reaction vessel purged with nitrogen, and a nitrogen gas was bubbled for 30 minutes while applying ultrasonic waves. 0.8 g of tris(dibenzylideneacetone)dipalladium and 0.5 g of 2,2'-bis (diphenylphosphino)-1,1'-binaphthyl were added thereto, and the mixture was heated and stirred for 16 hours at 110°C. After removing insoluble materials by filtration, the filtrate was concentrated. The residue was purified by column chromatography to obtain 25.4 g (yield of 75.6%) of a light-yellow viscous material of 4-(naphthalene-1-yl)phenyl-biphenyl-3-amine.

23.9 g of 4-(naphthalene-1-yl)phenyl-biphenyl-3-amine and 240 mL of dimethylformamide were added to the reaction vessel purged with nitrogen, and the mixture was cooled in an ice bath. 11.6 g of N-bromosuccinimide was added in portions over 30 minutes, and the mixture was stirred at 5°C for 4 hours. Water was added thereto, extraction with ethyl acetate was performed on the mixture to obtain an organic layer, and the organic layer was washed sequentially with water and saturated saline. The organic layer was dried with anhydrous magnesium sulfate, the desiccant was removed by filtration, and the filtrate was concentrated. The residue was purified by column chromatography to obtain 27.4 g (yield of 94.5%) of a yellow viscous material of 4-(naphthalene-1-yl)phenyl-6-bromobiphenyl-3-amine.

27.4 g of 4-(naphthalene-1-yl)phenyl-6-bromobiphenyl-3-amine, 16.8 g of 4-(1-naphthyl)phenylboronic acid, 270 mL of toluene, 90 mL of ethanol, and a solution obtained by dissolving 12.6 g of potassium carbonate in 90 mL of water were added to a reaction vessel purged with nitrogen, and a nitrogen gas was bubbled while applying ultrasonic waves. 0.7 g of tetrakis(triphenylphosphine)palladium was added thereto, and the mixture was heated and stirred at 72°C for 12 hours. The reaction solution was separated and was washed sequentially with water and saturated saline to obtain an organic layer, and the organic layer was dried with anhydrous magnesium sulfate. The desiccant was removed by filtration, and the filtrate was concentrated. The residue was purified by column chromatography to obtain 28.9 g (yield of 82.8%) of a yellow-white powder of {4"-(naphthalene-1-yl)-1,1':2',1''-terphenyl-5'-yl}-{4-(naphthalene-1-yl)phenyl}amine.

9.6 g of {4"-(naphthalene-1-yl)-1,1':2',1''-terphenyl-5'-yl}-{4-(naphthalene-1-yl)phenyl}amine, 5.2 g of 9-bromophenanthrene, 2.4 g of t-butoxy sodium, and 100 mL of toluene were added to a reaction vessel purged with nitrogen, and a nitrogen gas was bubbled while applying ultrasonic waves. 0.04 g of acetate palladium and 0.14 g of 50%(w/v) toluene solution of t-butylphosphine were added thereto, and the mixture was heated and stirred at 110°C for 11 hours. After removing insoluble materials by filtration, the filtrate was concentrated. The residue was purified by column chromatography to obtain 4.9 g (yield of 39%) of a white powder of {4-(naphthalene-1-yl)phenyl}-(phenanthrene-9-yl)-{4"-(naphthalene-1-yl)-1,1':2',1"-terphenyl-5'-yl}amine (Compound 1-11).

The structure of the obtained white powder was identified using NMR.

The following 39 hydrogen signals were detected by ¹H-NMR(CDCl₃₎.

δ(ppm)=8.73-8.86(2H), 8.28-8.33(1H), 8.06-8.12(1H), 7.86-7.95(7H), 7.62-7.78(4H), 7.17-7.78(24H).

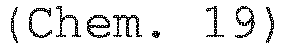

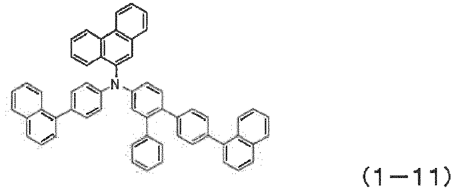

### (Example 2)

### <Synthesis of {4-(naphthalene-2-yl)phenyl}-(phenanthrene-9-yl)-(1,1':2',1"-terphenyl-5'-yl)amine (Compound 1-13)>

17.2 g of (1,1':2',1"-terphenyl-4'-yl)amine, 18.0 g of 2-(4-bromophenyl)naphthalene, 9.2 g of t-butoxy sodium, and 270 mL of toluene were added to a reaction vessel purged with nitrogen, and a nitrogen gas was bubbled while applying ultrasonic waves. 1.2 g of tris(dibenzylideneacetone)dipalladium and 0.8 g of 2,2'-bis(diphenylphosphino)-1,1'-binaphthyl were added thereto, and the mixture was heated and stirred at 110°C for 16 hours. After removing insoluble materials by filtration, the filtrate was heated and adsorption purification using activated clay or silica gel was performed thereon at 80°C to perform hot filtration. The filtrate was concentrated, and the residue was purified by recrystallization with toluene-heptane to obtain 24.3 g (yield of 85.4%) of a yellow-white powder of {4-(naphthalene-2-yl)phenyl}-(1,1':2',1''-terphenyl-5'-yl)amine.

24.3 g of {4-(naphthalene-2-yl)phenyl}-(1,1':2',1"-terphenyl-5'-yl)amine, 15.4 g of 9-bromophenanthrene, 10.4 g of t-butoxy sodium, and 240 mL of toluene were added to a reaction vessel purged with nitrogen, and a nitrogen gas was bubbled while applying ultrasonic waves. 0.2 g of acetate palladium and 0.4 g of 50%(w/v) toluene solution of t-butylphosphine were added thereto, and the mixture was heated at 110°C for 16 hours. After removing insoluble materials by filtration, the mixture was heated, and adsorption purification using activated clay or silica gel was performed thereon at 80°C to perform hot filtration. The filtrate was concentrated, and the residue was purified by recrystallization with toluene-heptane to obtain 30.9 g (yield of 91.2%) of a yellow-white powder of {4-(naphthalene-2-yl)phenyl}-(phenanthrene-9-yl)-(1,1':2',1"-terphenyl-5'-yl)amine (Compound 1-13).

The structure of the obtained yellow-white powder was identified using NMR.

The following 33 hydrogen signals were detected by ¹H-NMR (CDCl₃) .

δ(ppm)=8.76-8.84(2H), 8.23-8.27(1H), 8.05(1H), 7.84-7.94(5H), 7.47-7.78(9H), 7.34-7.38(4H), 7.12-7.29(11H).

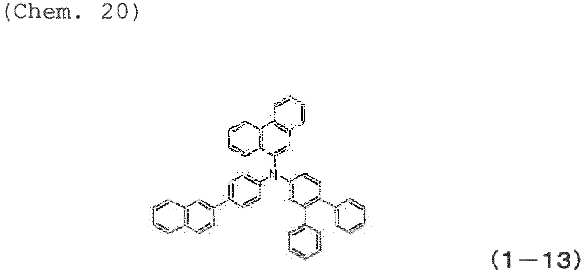

### (Example 3)

### <Synthesis of {4-(naphthalene-2-yl)phenyl}-(phenanthrene-9-yl)-{4"-(naphthalene-1-yl)-1,1':2',1"-terphenyl-5'-yl}amine (Compound 1-17)>

A similar operation was performed using {4"-(naphthalene-1-yl)-1,1':2',1''-terphenyl-5'-yl}-{4-(naphthalene-2-yl)phenyl}amine instead of {4"-(naphthalene-1-yl)-1,1':2',1"-terphenyl-5'-yl}-{4-(naphthalene-1-yl)phenyl}amine in Example 1 to obtain 4.9 g (yield of 40%) of a white powder of {4-(naphthalene-2-yl)phenyl}-(phenanthrene-9-yl)-{4"-(naphthalene-1-yl)-1,1':2',1"-terphenyl-5'-yl}amine (Compound 1-17).

The structure of the obtained white powder was identified using NMR.

The following 39 hydrogen signals were detected by ¹H-NMR(CDCl₃).

δ(ppm)=8.76-8.86(2H), 8.24-8.28(1H), 8.06(1H), 7.85-7.95(8H), 7.36-7.78(19H), 7.19-7.31(8H).

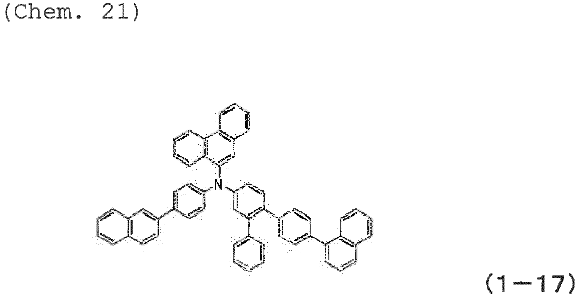

### (Example 4)

### <Synthesis of [1,1':4', 1"-terphenyl-4-yl]-(phenanthrene-9-yl)-(1,1':2',1"-terphenyl-5'-yl)amine (Compound 1-19)>

A similar operation was performed using [1,1':4', 1"-terphenyl-4-yl]-1,1':2',1"-terphenyl-5'-yl)amine instead of {4-(naphthalene-2-yl)phenyl}-(1,1':2',1"-terphenyl-5'-yl)amine in Example 2 to obtain 10.5 g (yield of 77.3%) of a yellow-white solid of [1,1':4', 1"-terphenyl-4-yl]-(phenanthrene-9-yl)-(1,1':2',1"-terphenyl-5'-yl)amine (Compound 1-19).

The structure of the obtained yellow-white solid was identified using NMR.

The following 35 hydrogen signals were detected by ¹H-NMR(CDCl₃).

δ(ppm)=8.75-8.83(2H), 8.19-8.21(1H), 7.85-7.87(1H), 7.80(1H), 7.46-7.74(14H), 7.07-7.41(16H).

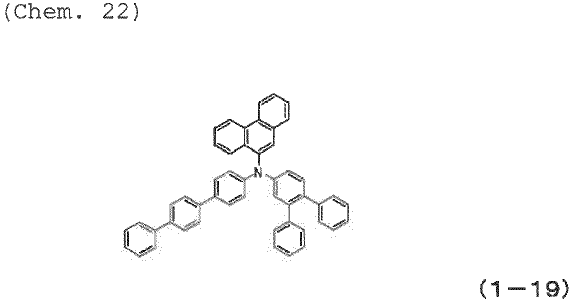

### (Example 5)

### <Synthesis of (biphenyl-4-yl)-(phenanthrene-9-yl)-{4''-(naphthalene-1-yl)-1,1':2',1"-terphenyl-4'-yl}amine (Compound 1-29)>

18.1g of 4-(naphthalene-1-yl)-1,1'-biphenyl-3'-amine, 13.0 g of 4-bromobiphenyl, 10.7 g of t-butoxy sodium, and 130 mL of toluene were added to a reaction vessel purged with nitrogen, and a nitrogen gas was bubbled while applying ultrasonic waves. 1.0 g of tris(dibenzylideneacetone)dipalladium and 10.5 g of 2,2'-bis(diphenylphosphino)-1,1'-binaphthy were added thereto, and the mixture was heated and stirred at 80°C for 16 hours. After removing insoluble materials by filtration, the filtrate was concentrated. The residue was purified by column chromatography to obtain 20.1 g (yield of 80.4%) of a pale yellow powder of {4-(naphthalene-1-yl)-1,1'-biphenyl-3'-yl}-biphenyl-4-amine.

19.5 g of {4-(naphthalene-1-yl)-1,1'-biphenyl-3'-yl}-biphenyl-4-amine and 200 mL of dimethylformamide were added to a reaction vessel purged with nitrogen, and the mixture was cooled in an ice bath. A solution obtained by dissolving 7.8 g of N-bromosuccinimide in 30 mL of dimethylformamide was added dropwise thereto over 1 hour, and the mixture was stirred at 5°C for 6 hours. Water was added thereto, the precipitated solid was collected by filtration, and the collected solid was washed with methanol to obtain 21.1 g (yield of 92.1%) of a yellow-white powder of {6'-bromo-4-(naphthalene-1-yl)-1,1'-biphenyl-3'-yl}-biphenyl-4-amine.

21.0 g of {6'-bromo-4-(naphthalene-1-yl)-1,1'-biphenyl-3'-yl}-biphenyl-4-amine, 5.8 g of phenylboronic acid, 170 mL of toluene, 80 mL of ethanol, and a solution obtained by dissolving 8.3 g of potassium carbonate in 60 mL of water were added to a reaction vessel purged with nitrogen, and a nitrogen gas was bubbled while applying ultrasonic waves. 0.9 g of tetrakis(triphenylphosphine)palladium was added thereto, and the mixture was heated and stirred at 70°C for 14 hours. The reaction solution was separated and was washed sequentially with water and saturated saline to obtain an organic layer, and the organic layer was dried with anhydrous magnesium sulfate. The desiccant was removed by filtration, and the filtrate was concentrated. The residue was purified by column chromatography to obtain 18.3 g (yield of 87.6%) of a yellow-white powder of {4"-(naphthalene-1-yl)-1,1':2',1"-terphenyl-3'-yl}-biphenyl-4-amine.

18.3 g of {4"-(naphthalene-1-yl)-1,1':2',1"-terphenyl-3-yl}-biphenyl-4-amine, 10.8 g of 9-bromophenanthrene, 5.0 g of t-butoxy sodium, and 180 mL of toluene were added to a reaction vessel purged with nitrogen, and a nitrogen gas was bubbled while applying ultrasonic waves. 0.16 g of acetate palladium and 0.6 g of 50%(w/v) toluene solution of t-butylphosphine were added thereto, and the mixture was stirred at 110°C for 3 hours. After removing insoluble materials by filtration, the filtrate was concentrated. The residue was purified by column chromatography to obtain 15.8 g (yield of 64.6%) of a yellow-white powder of (biphenyl-4-yl)-(phenanthrene-9-yl)-{4-(naphthalene-1-yl)-1,1':2',1"-terphenyl-5'-yl}amine (Compound 1-29).

The structure of the obtained yellow-white powder was identified using NMR.

The following 37 hydrogen signals were detected by ¹H-NMR(CDCl₃).

δ(ppm)=8.76-8.85(2H), 8.25-8.28(1H), 7.84-7.94(5H), 7.21-7.78(29H).

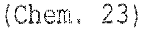

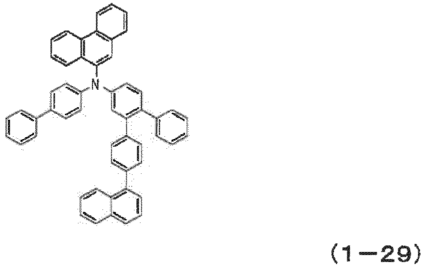

### (Example 6)

### <Synthesis of {4-(naphthalene-2-yl)phenyl}-(phenanthrene-9-yl)-{4"-(naphthalene-1-yl)-(1,1':2',1"-terphenyl)-4'-yl}amine (Compound 1-41)>

A similar operation was performed using {4"-(naphthalene-2-yl)phenyl}-{4"-(naphthalene-1-yl)-(1,1':2',1"-terphenyl)-4'-yl}amine instead of {4"-(naphthalene-1-yl)-1,1':2',1"-terphenyl-3-yl}-biphenyl-4-amine in Example 5 to obtain 8.9 g (yield of 74%) of a white powder of {4-(naphthalene-2-yl)phenyl}-(phenanthrene-9-yl)-{4"-(naphthalene-1-yl)-(1,1':2',1"-terphenyl)-4'-yl}amine(Compound 1-41).

The structure of the obtained white powder was identified using NMR.

The following 37 hydrogen signals were detected by¹H-NMR(CDCl₃).

δ(ppm)=8.75-8.85(2H), 8.22-8.26(1H), 8.05(1H), 7.83-7.93(5H), 7.16-7.78(28H).

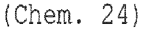

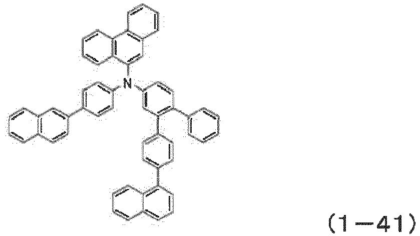

### (Example 7)

### <Synthesis of {4-(naphthalene-2-yl)phenyl}-(phenanthrene-9-yl)-{4"-(dibenzofuran-2-yl)-(1,1':2',1"-terphenyl)-4'-yl}amine (Compound 1-101)>

A similar operation was performed using {4"-(dibenzofuran-2-yl)phenyl}-{4"-(naphthalene-1-yl)-(1,1':2',1"-terphenyl)-4'-yl}amine instead of {4"-(naphthalene-1-yl)-1,1':2',1"-terphenyl-3-yl}-biphenyl-4-amine in Example 5 to obtain 3.6 g (yield of 46%) of a white powder of {4-(naphthalene-2-yl)phenyl}-(phenanthrene-9-yl)-{4"-(dibenzofuran-2-yl)-(1,1':2',1"-terphenyl)-4'-yl}amine (Compound 1-101).

The structure of the obtained white powder was identified using NMR.

The following 39 hydrogen signals were detected by ¹H-NMR(CDCl₃).

δ(ppm)=8.75-8.83(2H), 8.20-8.23(1H), 7.82-8.10(8H), 7.57-7.77(10H), 7.46-7.52(4H), 7.17-7.39(14H).

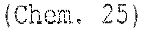

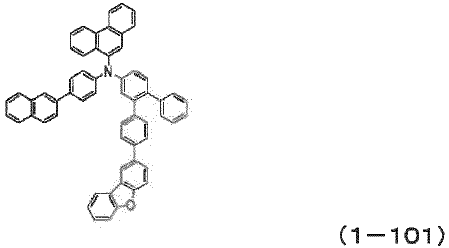

### (Example 8)

### <Synthesis of {4-(naphthalene-2-yl)phenyl}-(10-phenylphenanthrene-9-yl)-(1,1':2',1"-terphenyl-5'-yl)amine (Compound 1-105)>

A similar operation was performed using 9-bromo-10-phenylphenanthrene instead of 9-bromophenanthrene in Example 2 to obtain 3.6 g (yield of 46%) of a yellow-white powder of {4-(naphthalene-2-yl)phenyl}-(10-phenylphenanthrene-9-yl)-(1,1':2',1"-terphenyl-5'-yl)amine (Compound 1-105).

The structure of the obtained yellow-white powder was identified using NMR.

The following 37 hydrogen signals were detected by ¹H-NMR(CDCl₃).

δ(ppm)=8.85-8.89(2H), 8.18-8.22(1H), 8.00(1H), 7.83-7.87(3H), 7.71-7.78(3H), 7.45-7.63(7H), 6.86-7.32(20H) .

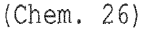

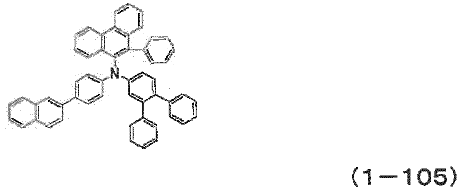

### (Example 9)

The glass transition point of the arylamine compound represented by the general formula (1) was measured using a high sensitivity differential scanning calorimeter(DSC3100SA manufactured by Bruker AXS GmbH).

| | Glass transition point |
|---|---|
| Compound of Example 1 | 137°C |
| Compound of Example 2 | 120°C |
| Compound of Example 3 | 137°C |
| Compound of Example 4 | 126°C |
| Compound of Example 5 | 135°C |
| Compound of Example 6 | 137°C |
| Compound of Example 7 | 149°C |
| Compound of Example 8 | 141°C |

The arylamine compound represented by the general formula (1) has a glass transition point of 100°C or higher, which indicates that the thin-film state is stable.

### (Example 10)

A deposition film having a film thickness of 100 nm was prepared on an ITO substrate by using the arylamine compound represented by the general formula (1), and the work function thereof was measured by an ionization potential measuring apparatus (PYS-202 manufactured by Sumitomo Heavy Industries, Ltd.).

| | Work function |
|---|---|
| Compound of Example 1 | 5.76 eV |
| Compound of Example 2 | 5.75 eV |
| Compound of Example 3 | 5.74 eV |
| Compound of Example 4 | 5.73 eV |
| Compound of Example 5 | 5.76 eV |
| Compound of Example 6 | 5.76 eV |
| Compound of Example 7 | 5.77 eV |
| Compound of Example 8 | 5.69 eV |

It can be seen that the arylamine compound represented by the general formula (1) has favorable a hole transport capacity because it exhibits a more suitable energy level than the work function that a general hole transport material such as NPD and TPD has, which is 5.4 eV.

### (Example 11)

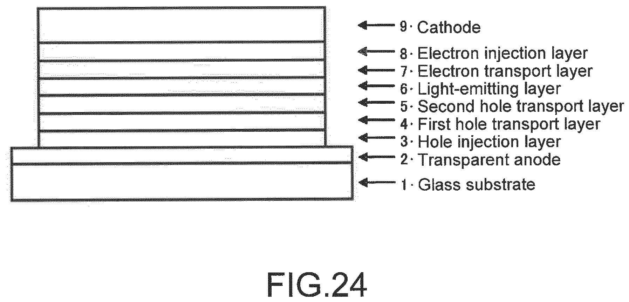

The organic EL device was prepared by depositing a hole injection layer 3, a first hole transport layer 4, a second hole transport layer 5, a light-emitting layer 6, an electron transport layer 7, an electron injection layer 8, and a cathode (aluminum electrode) 9 in the stated order on a transparent anode 2, which has been formed on a glass substrate 1 as an ITO electrode in advance, as shown in Fig. 24.

Specifically, after performing, in isopropyl alcohol for 20 minutes, ultrasonic cleaning on the glass substrate 1 on which ITO having a film thickness of 150 nm was deposited, the glass substrate 1 was dried for 10 minutes on a hot plate heated to 200°C. After that, UV ozone treatment was performed for 15 minutes, and then, this ITO-attached glass substrate was mounted in a vacuum deposition machine. The pressure in the vacuum deposition machine was reduced to 0.001 Pa or less. Subsequently, a film of an electron acceptor (Acceptor-1) having the following structural formula and the Compound (4-1) having the following structural formula was formed, as the hole injection layer 3, to have a film thickness of 5 nm and cover the transparent anode 2 by binary deposition at a deposition rate in which the ratio of the deposition rates of (Acceptor-1) and the Compound (4-1) was 3:97. As the first hole transport layer 4, a film of the Compound (4-1) having the following structural formula was formed on this hole injection layer 3 to have a film thickness of 45 nm. As the second hole transport layer 5, a film of the Compound (1-11) according to Example 1 was formed on this first hole transport layer 4 to have a film thickness of 10 nm. As the light-emitting layer 6, a film of the Compound (5-1) having the following structural formula and the Compound (EMH-1) having the following structural formula was formed on the second hole transport layer 5 to have a film thickness of 20 nm by binary deposition at a deposition rate in which the ratio of the deposition rates of the Compound (5-1) and the Compound (EMH-1) was 5:95. As the electron transport layer 7, a film of the Compound (3-125) having the following structural formula and the Compound (ETM-1) having the following structural formula was formed on this light-emitting layer 6 to have a film thickness of 30 nm by binary deposition at a deposition rate in which the ratio of the deposition rates of the Compound (3-125) and the Compound ETM-1 was 50:50. As the electron injection layer 8, a film of lithium fluoride was formed on this electron transport layer 7 to have a film thickness of 1 nm. Finally, aluminum was deposited thereon to have a thickness of 100 nm to form the cathode 9. The properties of the prepared organic EL device were measured at room temperature in the atmosphere. The measurement results of the light-emitting properties when a direct current voltage was applied to the prepared organic EL device were collectively shown in Table 1.

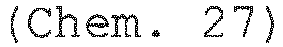

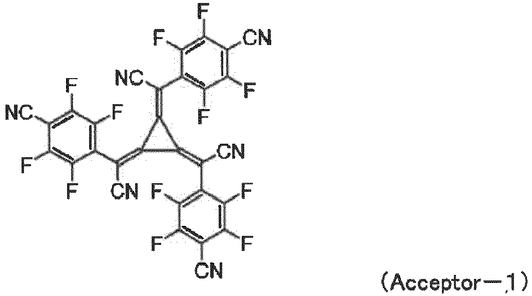

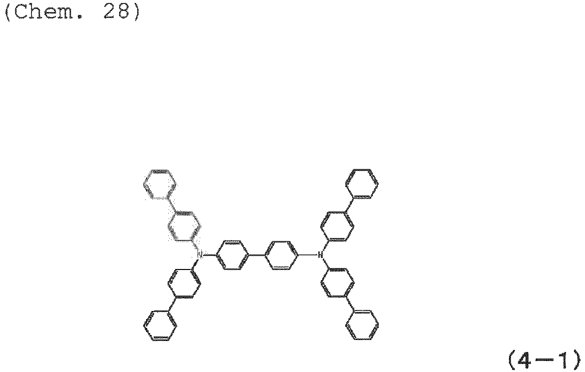

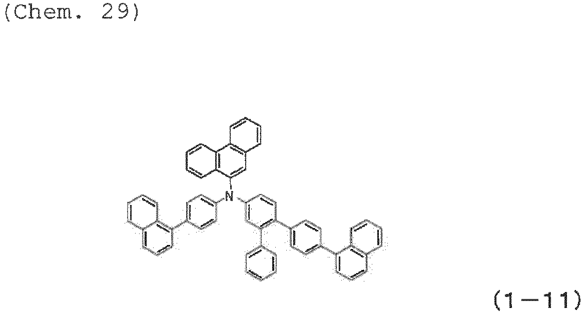

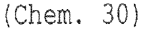

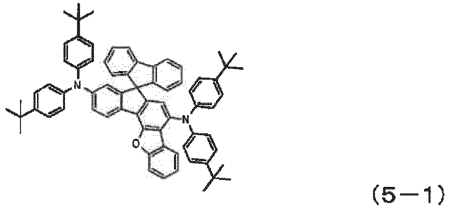

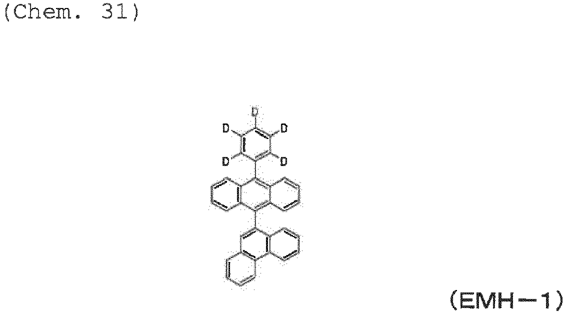

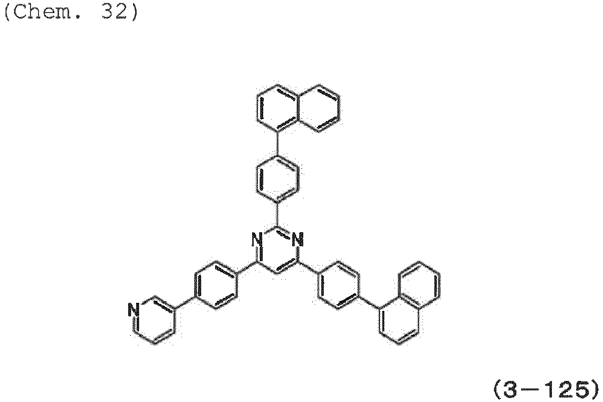

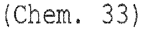

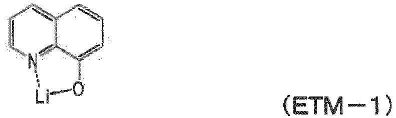

### (Example 12)

An organic EL device was prepared under conditions similar to those in Example 11 except that the Compound (1-13) according to Example 2 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

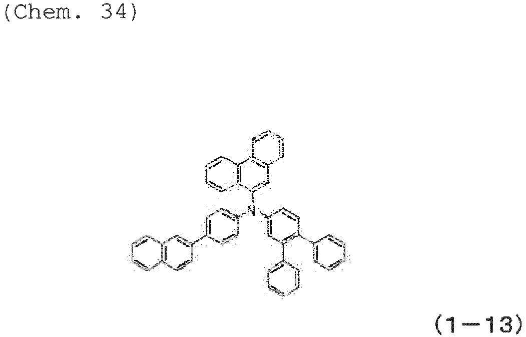

### (Example 13)

An organic EL device was prepared under conditions similar to those in Example 11 except that the Compound (1-17) according to Example 3 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

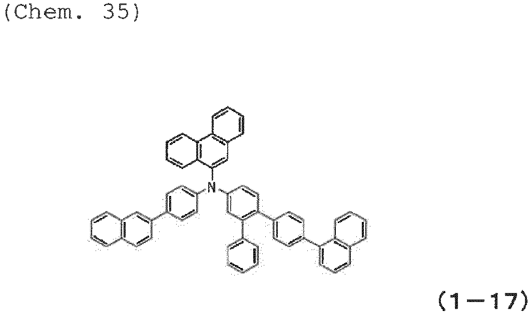

### (Example 14)

An organic EL device was prepared under conditions similar to those in Example 11 except that the Compound (1-19) according to Example 4 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

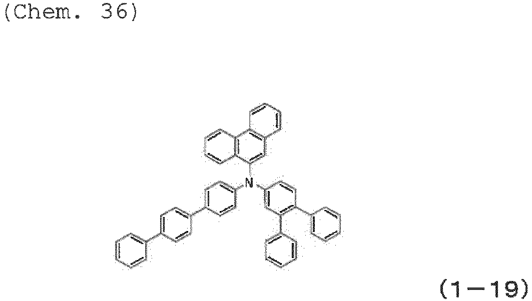

### (Example 15)

An organic EL device was prepared under conditions similar to those in Example 11 except that the Compound (1-29) according to Example 5 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

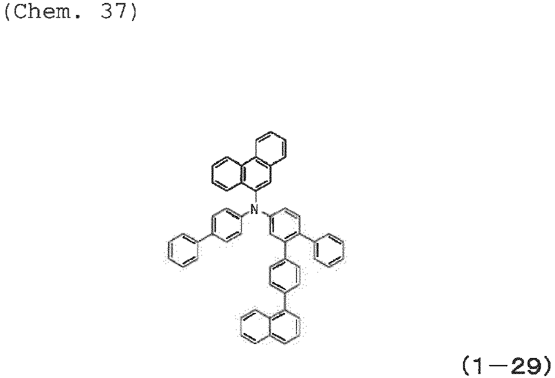

### (Example 16)

An organic EL device was prepared under conditions similar to those in Example 11 except that the Compound (1-41) according to Example 6 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

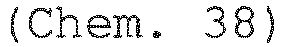

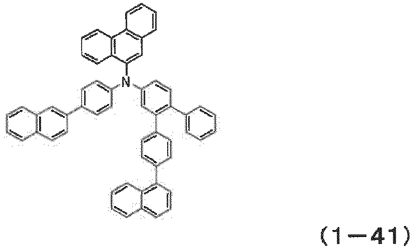

### (Example 17)

An organic EL device was prepared under conditions similar to those in Example 11 except that the Compound (1-101) according to Example 7 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

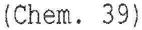

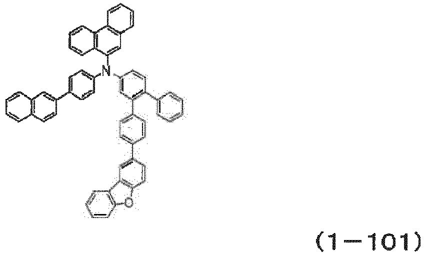

### (Example 18)

An organic EL device was prepared under conditions similar to those in Example 11 except that the Compound (1-105) according to Example 8 was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

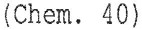

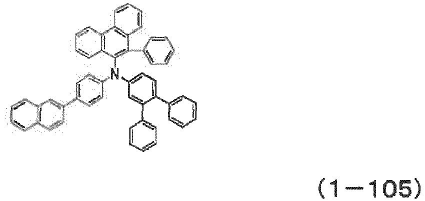

### [Comparative Example 1]

For comparison, an organic EL device was prepared under conditions similar to those in Example 11 except that the Compound (HTM-1) having the following structural formula was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

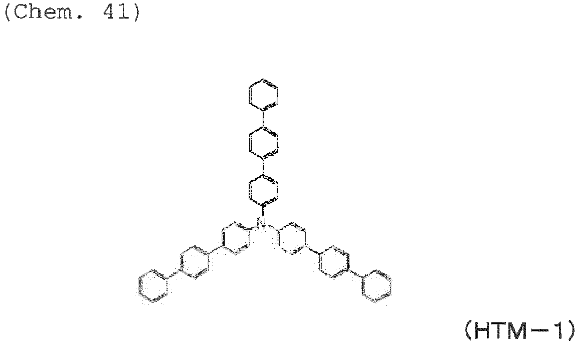

### [Comparative Example 2]

For comparison, an organic EL device was prepared under conditions similar to those in Example 11 except that the Compound (HTM-2) having the following structural formula was used as the material of the second hole transport layer 5 instead of the Compound (1-11) according to Example 1. The properties of the prepared organic EL device were measured at room temperature in the atmosphere. The measurement results of the light-emitting properties when a direct current voltage was applied to the prepared organic EL device were collectively shown in Table 1.

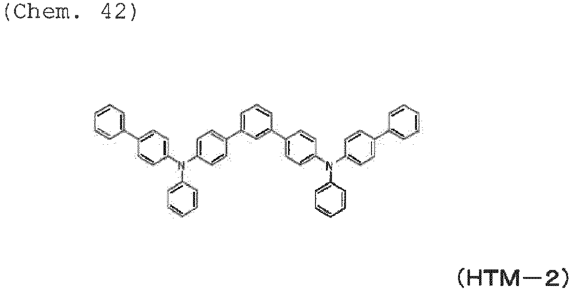

The device lifetime was measured using each of the organic EL devices prepared in Examples 11 to 18 and Comparative Examples 1 and 2, and the results were collectively shown in Table 1. The device lifetime was measured as the time until the light-emission luminance attenuated to 1900 cd/m² (corresponding to 95% in the case where the initial luminance was 100%: 95% attenuation) when constant current driving was performed with the light emission luminance (initial luminance) at the start of light emission set to 2000 cd/m².

**(Table 1)**

| | Second hole transport layer | Voltage[V] **(10mm/cm2)** | Luinance[cd/m2] **(10mm/cm2)** | Light emiss on efficency(cdA) **(10m/cm2)** | Power efficiency[lm/W] **10m/cm2)** | Device lifetime 95% attenuation |
|---|---|---|---|---|---|---|
| Example 11 | Compound 1-11 | **3.69** | **8.57** | **8.57** | **7.30** | **210** hours |
| Example 12: | Compound 1-13 | **3.68** | **811** | **8.11** | **6.92** | **294** hours |
| Example 13 | Compound 1-17 | **3.69** | **840** | **8.40** | **7.15** | **245** hours |
| Example 14 | Compound 1-19 | **3.68** | **822** | **8.22** | **7.02** | **233** hours |
| Example 15 | Compound 1-29 | **3.69** | **846** | **8.46** | **7.20** | **229** hours |
| Example 16 | Compound 1-41 | **3.68** | **819** | 8.19 | **6.99** | **285** hours |
| Example 17 | Compound 1-101 | **3.69** | **850** | **8.50** | **7.24** | **223** hours |
| Example 18 | Compound 1-105 | **3.68** | **815** | **8.15** | **6.95** | **238** hours |
| Comparative Example 1 | **HTM―1** | **3.66** | **751** | **7.51** | **6.45** | **160** hours |
| Comparative Example 2 | **HTM―2** | **3.65** | **770** | **7.70** | **6.63** | **120** hours |

As shown in Table 1, the light emission efficiency when a current having a current density of 10 mA/cm² was caused to flow was high, i.e., 8.11 to 8.57 cd/A in the organic EL devices according to Examples 11 to 18 as compared with 7.51 to 7.70 cd/A of the organic EL devices according to Comparative Examples 1 and 2. Further, also the power efficiency of the organic EL devices according to Examples 11 to 18 was high, i.e., 6.92 to 7.30 lm/W as compared with 6.45 to 6.63 lm/W of the organic EL devices according to Comparative Examples 1 and 2. Further, it can be seen that the device lifetime (95% attenuation) was extended to 210 to 294 hours in the organic EL devices according to Examples 11 to 18 as compared with 120 to 160 hours of the organic EL devices according to Comparative Examples 1 and 2.

As is clear from the results described above, it has been found that since the organic EL device according to the present invention uses an arylamine compound having a high mobility of holes and excellent electron blocking performance, an organic EL device having higher light emission efficiency and a longer lifetime as compared with those of the existing organic EL device can be realized.

### Industrial Applicability

The organic EL device according to the present invention, which uses an arylamine compound having a specific structure, has improved light emission efficiency and an improved durability, and, for example, it has become possible to expand to home appliances and lighting applications.

- 1: glass substrate
- 2: transparent anode
- 3: hole injection layer
- 4: first hole transport layer
- 5: second hole transport layer
- 6: light-emitting layer
- 7: electron transport layer
- 8: electron injection layer
- 9: cathode

## Claims

1. An organic electroluminescence device, comprising:
at least an anode, a hole transport layer, a light-emitting layer, an electron transport layer, and a cathode in the stated order, the organic electroluminescence device being **characterized in that**
the hole transport layer includes an arylamine compound represented by the following general formula (1) . 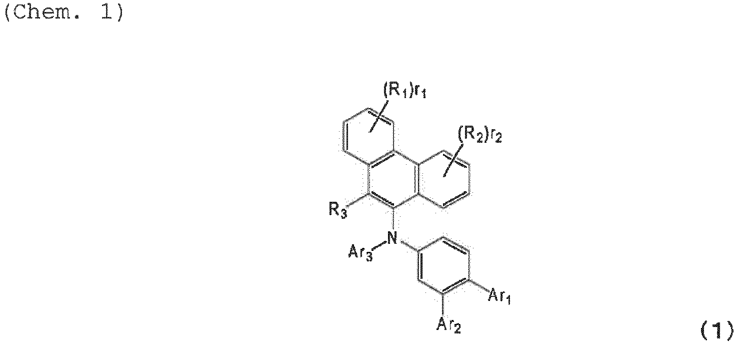
(In the formula, R₁ and R₂ each represent a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. R₃ represents a hydrogen atom, a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. Ar₁ to Ar₃ each represent a substituted or unsubstituted aromatic hydrocarbon group, a substituted or unsubstituted aromatic heterocyclic group, or a substituted or unsubstituted fused polycyclic aromatic group. r₁ and r₂ may be the same as or different from each other and each represent an integer of 0 to 4.)

2. The organic electroluminescence device according to claim 1, **characterized in that**
the arylamine compound is represented by the following general formula (1a). 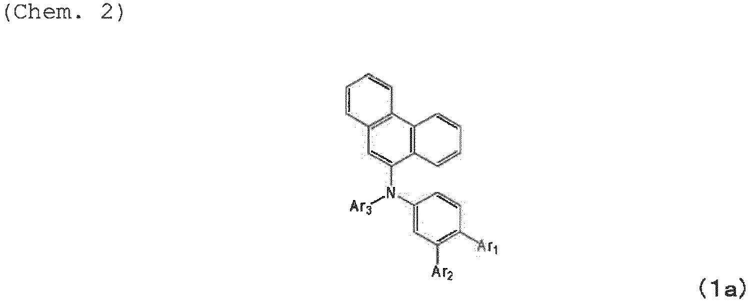 (In the formula, Ar₁ to Ar₃ are as shown in the general formula (1).)

3. The organic electroluminescence device according to claim 2, **characterized in that**
the Ar3 is a substituted or unsubstituted phenyl group.

4. The organic electroluminescence device according to any one of claims 1 to 3, **characterized in that**
the electron transport layer includes a compound having a pyrimidine ring structure represented by the following general formula (2). 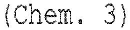 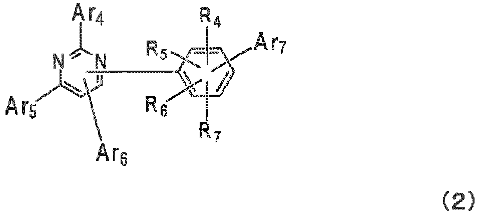 (In the formula, Ar₄ represents a substituted or unsubstituted aromatic hydrocarbon group or a substituted or unsubstituted fused polycyclic aromatic group, Ar₅ and Ar₆ may be the same as or different from each other and each represent a hydrogen atom, a substituted or unsubstituted aromatic hydrocarbon group, or a substituted or unsubstituted fused polycyclic aromatic group, Ar₇ represents a substituted or unsubstituted aromatic heterocyclic group, and R₄ to R₇ may be the same as or different from each other and each represent a hydrogen atom, a deuterium atom, a fluorine atom, a chlorine atom, a cyano group, a trifluoromethyl group, a linear or branched alkyl group having 1 to 6 carbon atoms, a substituted or unsubstituted aromatic hydrocarbon group, a substituted or unsubstituted aromatic heterocyclic group, or a substituted or unsubstituted fused polycyclic aromatic group. Here, there is no case that both Ar₅ and Ar₆ are hydrogen atoms.)

5. The organic electroluminescence device according to any one of claims 1 to 4, **characterized in that**
the hole transport layer has a two-layer structure of a first hole transport layer and a second hole transport layer, and
the second hole transport layer is adjacent to the light-emitting layer and includes the arylamine compound.

6. The organic electroluminescence device according to claim 5, **characterized in that**
the first hole transport layer includes a triphenylamine derivative different from the arylamine compound included in the second hole transport layer, and
the triphenylamine derivative is a compound that has a molecular structure in which two triphenylamine skeletons are linked to each other by a single bond or a divalent hydrocarbon group and has two to six triphenylamine skeletons as a whole molecule.

7. The organic electroluminescence device according to claim 5 or 6, **characterized in that**
the triphenylamine derivative included in the first hole transport layer is a compound represented by the following general formula (3). 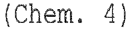 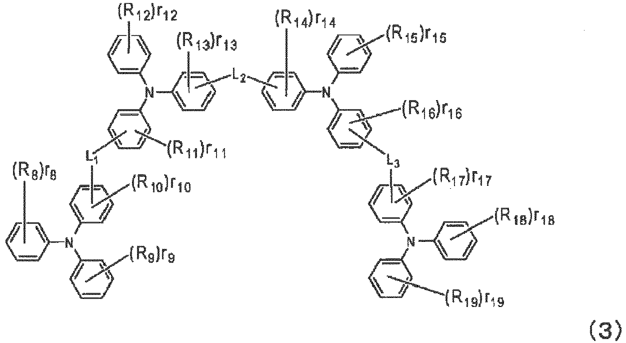 (In the formula, R₈ to R₁₉ each represent a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₈ to r₁₉ may be the same as or different from each other, r₈, r₉, r₁₂, r₁₅, r₁₈, and r₁₉ each represent an integer of 0 or 1 to 5, and r₁₀, r₁₁, r₁₃, r₁₄, r₁₆, and r₁₇ each represent an integer of 0 or 1 to 4. In a case where r₈, r₉, r₁₂, r₁₅, r₁₈, and r₁₉ each represent an integer of 2 to 5 or r₁₀, r₁₁, r₁₃, r₁₄, r₁₆, and r₁₇ each represent an integer of 2 to 4, a plurality of R₈, a plurality of R₉, a plurality of R₁₀, a plurality of R₁₁, a plurality of R₁₂, a plurality of R₁₃, a plurality of R₁₄, a plurality of R₁₅, a plurality of R₁₆, a plurality of R₁₇, a plurality of R₁₈, or a plurality of R₁₉ bonded to the same benzene ring may be the same as or different from each other and may be bonded to each other via a single bond, a substituted or unsubstituted methylene group, an oxygen atom, or a sulfur atom to form a ring. L₁, L₂, and L₃ may be the same as or different from each other and each represent a divalent group represented by one of the following structural formulae (B) to (G) or a single bond.) 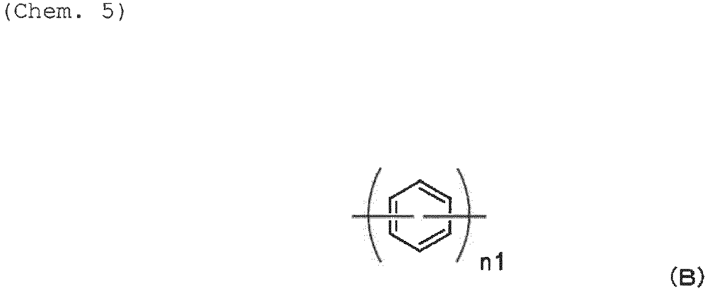 (In the formula, n1 represents an integer of 1 to 3.) 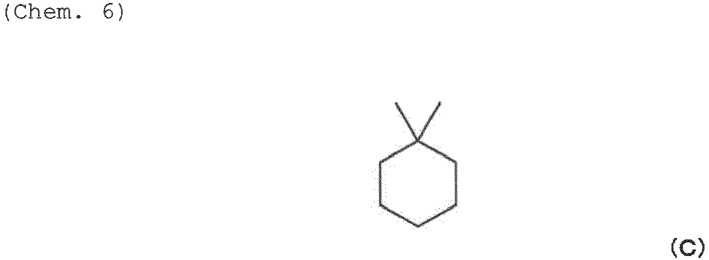 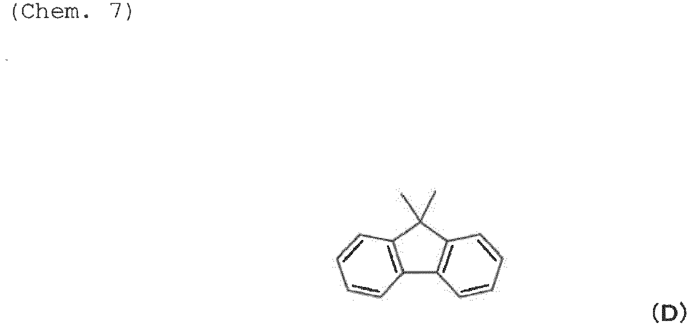 (Chem. 7)
-CH₂- (E)

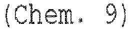

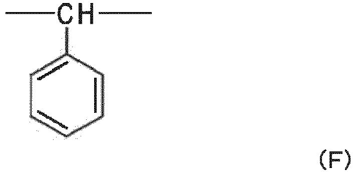

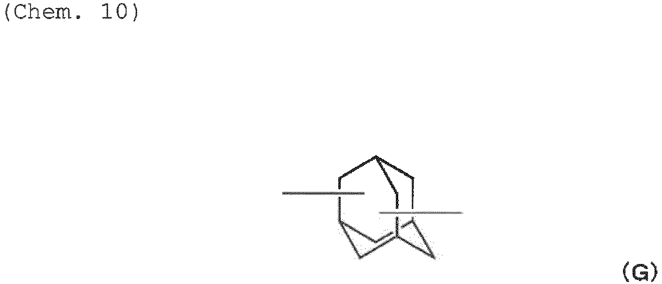

8. The organic electroluminescence device according to claim 5 or 6, **characterized in that**
the triphenylamine derivative included in the first hole transport layer is a compound represented by the following general formula (4). 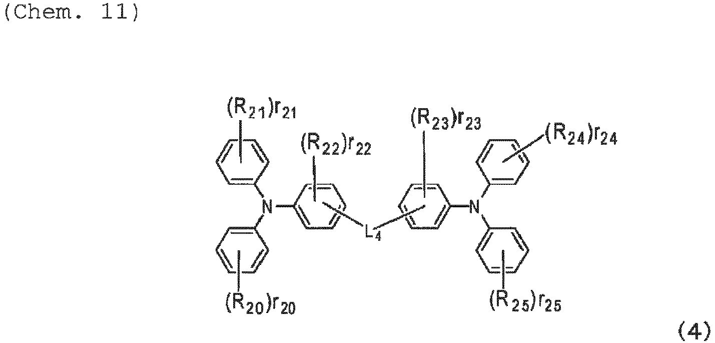 (In the formula, R₂₀ to R₂₅ each represent a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. r₂₀ to r₂₅ may be the same as or different from each other, r₂₀, r₂₁, r₂₄, and r₂₅ each represent an integer of 0 or 1 to 5, and r₂₂ and r₂₃ each represent an integer of 0 or 1 to 4. In a case where r₂₀, r₂₁, r₂₄, and r₂₅ each represent an integer of 2 to 5 or r₂₂ and r₂₃ each represent an integer of 2 to 4, a plurality of R₂₀, a plurality of R₂₁, a plurality of R₂₂, a plurality of R₂₃, a plurality of R₂₄, or a plurality of R₂₅ bonded to the same benzene ring may be the same as or different from each other and may be bonded to each other via a single bond, a substituted or unsubstituted methylene group, an oxygen atom, or a sulfur atom to form a ring. L₄ represents a divalent group represented by one of the following structural formulae (B) to (G) or a single bond.) 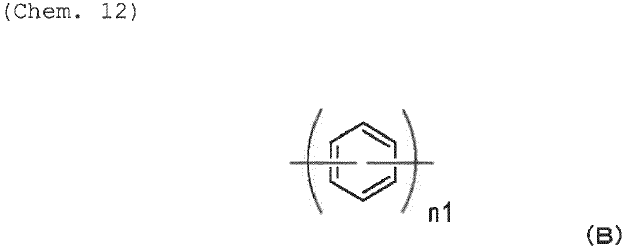 (In the formula, n1 represents an integer of 1 to 3.) 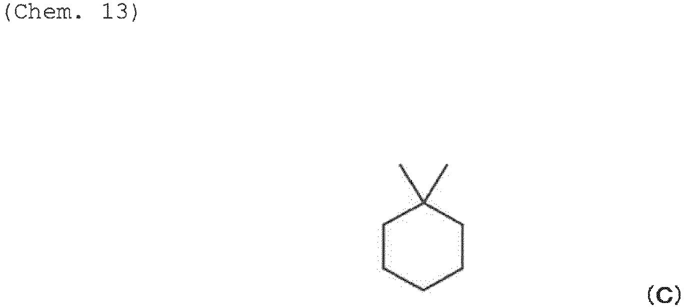 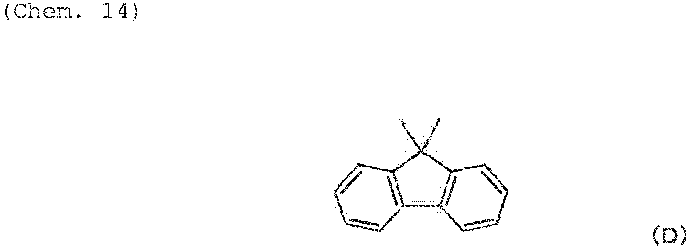 (Chem. 15)
-CH₂- (E)

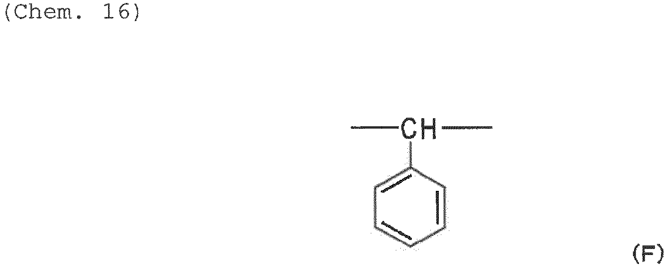

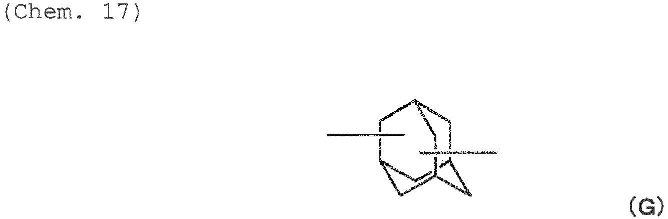

9. The organic electroluminescence device according to any one of claims 1 to 8, **characterized in that**
the light-emitting layer includes a blue luminescent dopant.

10. The organic electroluminescence device according to claim 9, **characterized in that**
the blue luminescent dopant is a pyrene derivative having a pyrene skeleton in a molecule.

11. The organic electroluminescence device according to claim 9, **characterized in that**
the blue luminescent dopant is an amine derivative having a fused ring structure represented by the following general formula (5). 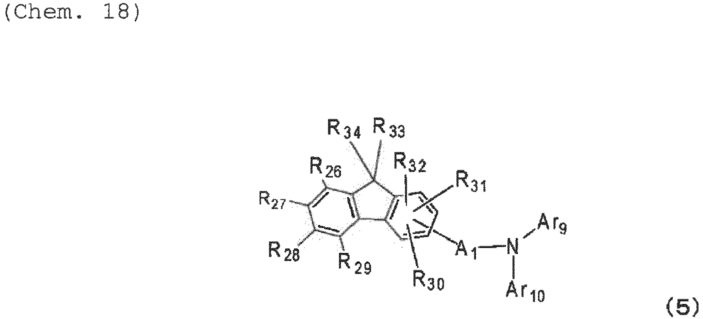 (In the formula, A₁ represents a divalent group of a substituted or unsubstituted aromatic hydrocarbon, a divalent group of a substituted or unsubstituted aromatic heterocyclic ring, a divalent group of a substituted or unsubstituted fused polycyclic aromatic, or a single bond, and Arg and Ar₁₀ may be the same as or different from each other, each represent a substituted or unsubstituted aromatic hydrocarbon group, a substituted or unsubstituted aromatic heterocyclic group, or a substituted or unsubstituted fused polycyclic aromatic group, and may be bonded to each other via a single bond, a substituted or unsubstituted methylene group, an oxygen atom, or a sulfur atom to form a ring. R₂₆ to R₂₉ may be the same as or different from each other and each represent a hydrogen atom, a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, a substituted or unsubstituted aryloxy group, or a disubstituted amino group substituted with a group selected from the group consisting of an aromatic hydrocarbon group, an aromatic heterocyclic group, and a fused polycyclic aromatic group, and R₂₆ to R₂₉ may be bonded to each other via a single bond, a substituted or unsubstituted methylene group, an oxygen atom, or a sulfur atom to form a ring and may be bonded to the
benzene ring to which R₂₆ to R₂₉ are bonded via a linking group such as a substituted or unsubstituted methylene group, an oxygen atom, a sulfur atom, and a monosubstituted amino group to form a ring. R₃₀ to R₃₂ may be the same as or different from each other and each represent a hydrogen atom, a deuterium atom, a fluorine atom, a chlorine atom, a cyano group, a nitro group, a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a linear or branched alkyloxy group having 1 to 6 carbon atoms which may have a substituent group, a cycloalkyloxy group having 5 to 10 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and R₃₀ to R₃₂ may be bonded to each other via a single bond, a substituted or unsubstituted methylene group, an oxygen atom, or a sulfur atom to form a ring and may be bonded to the benzene ring to which R₃₀ to R₃₂ are bonded via a linking group such as a substituted or unsubstituted methylene group, an oxygen atom, a sulfur atom, and a monosubstituted amino group to form a ring. R₃₃ and R₃₄ may be the same as or different from each other and each represent a linear or branched alkyl group having 1 to 6 carbon atoms which may have a substituent group, a cycloalkyl group having 5 to 10 carbon atoms which may have a substituent group, a linear or branched alkenyl group having 2 to 6 carbon atoms which may have a substituent group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group, and R₃₃ and R₃₄ may be bonded to each other via a linking group such as a single bond, a substituted or unsubstituted methylene group, an oxygen atom, a sulfur atom, and a monosubstituted amino group to form a ring.)

12. The organic electroluminescence device according to any one of claims 1 to 11, **characterized in that**
the light-emitting layer includes an anthracene derivative having an anthracene skeleton in a molecule.

13. The organic EL device according to claim 12, **characterized in that**
the light-emitting layer includes a host material that is an anthracene derivative.
